(19) Europäisches Patentamt
European Patent Office
Office européen des brevets

(11) **EP 4 216 223 A1**

(12) **EUROPEAN PATENT APPLICATION**

(43) Date of publication:
**26.07.2023 Bulletin 2023/30**

(51) International Patent Classification (IPC):
***G16B 20/00*** *(2019.01)*    ***G16B 40/10*** *(2019.01)*

(21) Application number: **22152865.6**

(22) Date of filing: **24.01.2022**

(52) Cooperative Patent Classification (CPC):
**G16B 20/00; G16B 40/10**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Dabrowski, Piotr Wojciech
13599 Berlin (DE)**

(72) Inventors:
• **Dabrowski, Piotr Wojciech
13599 Berlin (DE)**
• **Ulrich, Jens-Uwe
15745 Wildau (DE)**
• **Renard, Bernhard
14055 Berlin (DE)**

(74) Representative: **Hertin und Partner
Rechts- und Patentanwälte PartG mbB
Kurfürstendamm 54/55
10707 Berlin (DE)**

(54) **NANOPORE BIOPOLYMER SEQUENCING METHOD COMPRISING ADAPTIVE SAMPLING**

(57) The invention relates to a method for determining the presence of one or more target sequences in a biopolymer-comprising sample, comprising: providing a nanopore sequencing library generated from the biopolymer-comprising sample, subjecting the sequencing library to a nanopore sequencing process comprising the following analysis steps: i.) obtaining a biopolymer sequence by sequencing 10-1500 consecutive building blocks of a biopolymer fragment in a nanopore of a nanopore sequencing device, ii.) comparing the biopolymer sequence obtained in step i. to sequences comprised within a decoy sequence database and/or comparing the biopolymer sequence obtained in step i. to the one or more target sequences comprised within a target sequence database by applying a probabilistic data structure approach that utilizes hash functions, subsequences and bit arrays for sequence comparison, wherein a) if the sequence obtained in step i. matches to at least one decoy sequence comprised by the decoy sequence database but not to the sequences comprised by the target database, the sequencing of the respective biopolymer fragment is aborted and the biopolymer fragment is discarded from the nanopore, or b) if the sequence obtained in step i. matches to at least one target sequence comprised by the target sequence database but not to the sequences comprised by the decoy sequence database the presence of the target sequence of the target sequence database with the highest shared sequence identity to the obtained sequence is determined.

Fig. 2

## Description

The invention relates to a method for determining the presence of one or more target sequences in a biopolymer-comprising sample, comprising: providing a nanopore sequencing library generated from the biopolymer-comprising sample, subjecting the sequencing library to a nanopore sequencing process comprising the following analysis steps: i.) obtaining a biopolymer sequence by sequencing 10-1500 consecutive building blocks of a biopolymer fragment in a nanopore of a nanopore sequencing device, ii.) comparing the biopolymer sequence obtained in step i. to sequences comprised within a decoy sequence database and/or comparing the biopolymer sequence obtained in step i. to the one or more target sequences comprised within a target sequence database by applying a probabilistic data structure approach that utilizes hash functions, subsequences and bit arrays for sequence comparison, wherein a) if the sequence obtained in step i. matches to at least one decoy sequence comprised by the decoy sequence database but not to the sequences comprised by the target database, the sequencing of the respective biopolymer fragment is aborted and the biopolymer fragment is discarded from the nanopore, or b) if the sequence obtained in step i. matches to at least one target sequence comprised by the target sequence database but not to the sequences comprised by the decoy sequence database the presence of the target sequence of the target sequence database with the highest shared sequence identity to the obtained sequence is determined.

## BACKGROUND OF THE INVENTION

In medicine, agriculture, livestock farming and in environmental research fast and easy detection of microbes and pathogens is of high relevance. At present, there are two main options accessible for pathogen detection, 1. specific detection by PCR or pathogen cultivation, and 2. unspecific DNA or RNA sequencing of the sample material using high-throughput sequencing (HTS) technologies such as Illumina. In both cases, however, the analyzed samples must first be brought to the laboratory and processed in several manual steps until a result is available. This can take several days and might delay, for example, the prescription of a suitable antibiotic to a patient or an animal. Point-of-care (POC) or on-site tests are only available for few pathogens and are mostly based on antibody detection in a subject's blood. Means and methods for direct pathogen detection at the POC or on-site are therefore urgently needed.

Nanopore sequencers enable the user to sequence only DNA molecules of interest by rejecting other sequences from individual pores (adaptive sampling). This feature allows enriching low-abundant sequences by depleting overrepresented ones in-silico.

During the last decade, the invention of nanopore sequencing instruments has democratized DNA sequencing in various aspects (Mikheyev and Tin 2014; Leggett and Clark 2017). For example, the small MinION devices of Oxford Nanopore Technologies provide the possibility to sequence a sample at the place of its origin, without the need to ship the sample to a laboratory (Sim and B Chapman 2019; Runtuwene et al. 2019). This point-of-care or on-site sequencing ability makes nanopore sequencing attractive for applications such as pathogen detection in a clinical setting and in the field (Mongan et al. 2020; Quick et al. 2016). It also can shorten the time to detect pathogens or antimicrobial resistance (AMR) genes when using it for point-of-care testing.

While the size of the device and the easier and faster sample preparation are clear advantages, nanopore sequencing still lags the base quality of sequencing-by-synthesis instruments. However, recent improvements in base calling algorithms showed per read accuracy exceeding 90% (Rang et al. 2018; Wick et al. 2019). Oxford Nanopore Technologies (ONT) even claims to boost per read accuracy up to 98% with their latest R10.3 pore version (Oxford Nanopore Technologies 2020).

Another exciting feature of ONT's instruments is the sequencing of DNA molecules in a targeted fashion. Oxford Nanopore provides an Application Programming Interface (API) that enables receiving electrical currents, measured while the molecule transverses the pore (Loose et al. 2016). These signals can be translated into sequence space and analyzed in real-time. An uninteresting DNA molecule currently located in a pore can be ejected by sending an "unblock"-message back to the control software. This message leads the sequencer to reverse the voltage across the pore, causing the molecule to exit the pore in the reverse direction. The primary requirement for such a live depletion system is that the software making ejection decisions can keep up with the sequencing speed for up to 512 nanopores that concurrently sequence DNA molecules on a MinION sequencer.

Two recent publications describe the implementation of such systems for specific settings. Payne, Holmes, Clarke, et al. combined Oxford Nanopore's Guppy base caller (Wick et al. 2019) with the minimap2 read aligner (Li 2018) in their Readfish workflow to make ejection decisions after mapping the reads to a reference genome in real-time. Kovaka et al. (Kovaka et al. 2021) skipped the base-calling step and performed ejection decisions directly on nanopore current signals. While the latter is designed to run on a general-purpose CPU, it cannot handle large human size reference genomes. In contrast, Readfish can handle larger references but needs GPUs and an additional base-calling server for real-time base calling.

Furthermore, the usage of minimap2 (Li 2018) for read classification is not optimal. In their study, Payne, Holmes, Clarke, et al. showed that only 83% of target reads were correctly classified for rejection after 0.8 seconds of sequencing. Further, missed mappings to repetitive regions of the reference genome can lead to delayed classifications when longer parts of the DNA molecule have to be sequenced to make a rejection decision. Both lower sensitivity and classification delay will cause decreased enrichment of clinically relevant sequences of undetected pathogens or antibiotic resistance markers.

With RUBRIC (Edwards et al. 2019), there exists another tool that enables selective nanopore sequencing. In contrast to Readfish, it uses Nanonet for online base calling and the Last aligner (Kiefbasa et al. 2011) for read classification. However, there are two concerns regarding RUBRIC. First, Nanonet is outdated and no longer supported or offered by Oxford Nanopore, and second, it does not work with newer versions of the ReadUntil API (Loose et al. 2016).

In view of these drawbacks of existing solutions, the problem underlying the present invention was to provide a method with an improved read classification approach that can make fast and more accurate decisions regarding the continuation or abortion of the sequencing at a nanopore during nanopore sequencing, also for large or repetitive sequences.

Additionally, in-field biologist have rarely on-site access to equipment comprising graphics processing units (GPUs), that are required for state-of-the-art live base-calling, or to extensive computational resources that are required for most of state-of-the-art methods for nanopore sequencing analysis. Therefore, it was another objective to provide a method that can also be performed on CPUs and standard computational hardware that overcomes the drawbacks of known technologies described above.

## SUMMARY OF THE INVENTION

The technical object is to provide improved means for nanopore sequencing analysis comprising adaptive sampling and sequence analysis, which can be performed on standard computational hardware.

This problem is solved by the features of the independent claims. Preferred embodiments of the present invention are provided by the dependent claims.

The invention therefore relates to a method for determining the presence of one or more target sequences in a biopolymer-comprising sample, comprising:

> providing a nanopore sequencing library generated from the biopolymer-comprising sample,
> subjecting the sequencing library to a nanopore sequencing process comprising the following analysis steps:

>> i. obtaining a biopolymer sequence by sequencing 10-1500 consecutive building blocks of a biopolymer fragment in a nanopore of a nanopore sequencing device,
>> ii. comparing the biopolymer sequence obtained in step i. to sequences comprised within a decoy sequence database and/or comparing the biopolymer sequence obtained in step i. to the one or more target sequences comprised within a target sequence database by applying a probabilistic data structure approach that utilizes hash functions, subsequences and bit arrays for sequence comparison,
>> wherein

>>> a. if the sequence obtained in step i. matches to at least one decoy sequence comprised by the decoy sequence database but not to the sequences comprised by the target database, the sequencing of the respective biopolymer fragment is aborted and the biopolymer fragment is discarded from the nanopore, or

>>> b. if the sequence obtained in step i. matches to at least one target sequence comprised by the target sequence database but not to the sequences comprised by the decoy sequence database, the presence of the target sequence of the target sequence database with the highest shared sequence identity to the obtained sequence is determined, or

>>> c. if the sequence obtained in step i. neither matches to at least one sequence comprised by the decoy sequence database nor to at least one sequence comprised by the target sequence database, or

>>>> if the sequence obtained in step i. matches to at least one sequence comprised by both the decoy sequence database and the target sequence database, the sequencing of the biopolymer fragment in the nanopore of step i. is continued for a defined length of building blocks, preferably at least 100, or alternatively until the end of the fragment, resulting in an elongated sequence, and the obtained elongated sequence is compared to sequences comprised within the decoy sequence database and/or to sequences comprised within the target sequence database according to step ii.,

wherein one of alternative steps ii. a., b. or c. is performed,

iii. optionally determining the presence of one or more target sequences in the sample according to step ii.b.

The invention also relates to a method for determining the presence of one or more target sequences in a biopolymer-comprising sample, comprising:

providing a nanopore sequencing library generated from the biopolymer-comprising sample,

subjecting the sequencing library to a nanopore sequencing process comprising the following analysis steps:

i. obtaining a biopolymer sequence by sequencing 10-1500 consecutive building blocks of a biopolymer fragment in a nanopore of a nanopore sequencing device,

ii. comparing the biopolymer sequence obtained in step i. to sequences comprised within a decoy sequence database and/or comparing the biopolymer sequence obtained in step i. to the one or more target sequences comprised within a target sequence database by applying a probabilistic data structure approach that utilizes hash functions, subsequences and bit arrays for sequence comparison, wherein

a. if the sequence obtained in step i. matches to at least one decoy sequence comprised by the decoy sequence database but not to the sequences comprised by the target database, the sequencing of the respective biopolymer fragment is aborted and the biopolymer fragment is discarded from the nanopore, or

b. if the sequence obtained in step i. matches to at least one target sequence comprised by the target sequence database but not to the sequences comprised by the decoy sequence database the presence of the target sequence of the target sequence database with the highest shared sequence identity to the obtained sequence is determined, and preferably the sequencing of the biopolymer fragment is continued, preferably until the end of the biopolymer fragment is reached and a final sequence is obtained, or

c. if the sequence obtained in step i. neither matches to at least one sequence comprised by the decoy sequence database nor to at least one sequence comprised by the target sequence database, or

if the sequence obtained in step i. matches to at least one sequence comprised by both the decoy sequence database and the target sequence database,

the sequencing of the biopolymer fragment in the nanopore of step i. is continued for a defined length of building blocks, preferably at least 100 building blocks, or alternatively until the end of the fragment, resulting in an elongated sequence,

wherein the obtained elongated sequence is compared to sequences comprised within the decoy sequence database and/or to sequences comprised within the target sequence database according to step ii., wherein one of alternative steps ii. a., b. or c. is performed,

iii. optionally determining the presence of one or more target sequences in the sample according to step ii.b.

In preferred embodiments of the present invention the biopolymer is selected from the group comprising nucleic acids and polypeptides. In some preferred embodiments the biopolymer is a nucleic acid. In other preferred embodiments the biopolymer is a polypeptide.
In specific embodiments the invention therefore relates to a method for determining the presence of one or more target sequences in a nucleic acid-comprising sample, comprising:

providing a nanopore sequencing library generated from the nucleic acid-comprising sample,

subjecting the sequencing library to a nanopore sequencing process comprising the following analysis steps:

i. sequencing 10-1500 consecutive nucleotides of a nucleic acid fragment in a nanopore of a nanopore sequencing device,

ii. comparing the nucleotide sequence obtained in step i. to sequences comprised within a decoy sequence database and/or comparing the nucleotide sequence obtained in step i. to the one or more target sequences comprised within a target sequence database by applying a probabilistic data structure approach that utilizes hash functions, subsequences and bit arrays for sequence comparison,
wherein

a. if the sequence obtained in step i. matches to at least one decoy sequence comprised by the decoy sequence database but not to the sequences comprised by the target database, the sequencing of the respective nucleic acid fragment is aborted and the nucleic acid fragment is discarded from the nanopore, or

b. if the sequence obtained in step i. matches to at least one target sequence comprised by the target sequence database but not to the sequences comprised by the decoy sequence database the presence of the target sequence of the target sequence database with the highest shared sequence identity to the obtained sequence is determined, and preferably the sequencing of the nucleic acid fragment is continued, preferably until the end of the nucleic acid fragment is reached and a final sequence is obtained, or

c. if the sequence obtained in step i. neither matches to at least one sequence comprised by the decoy sequence database nor to at least one sequence comprised by the target sequence database, or

if the sequence obtained in step i. matches to at least one sequence comprised by both the decoy sequence database and the target sequence database,

the sequencing of the nucleic acid fragment in the nanopore of step i. is continued for a defined length of nucleotides, preferably at least 100 nucleotides, or alternatively until the end of the fragment, resulting in an elongated sequence,

and the obtained elongated sequence is compared to sequences comprised within the decoy sequence database and/or to sequences comprised within the target sequence database according to step ii., wherein one of alternative steps ii. a., b. or c. is performed.

iii. optionally determining the presence of one or more target sequences in the sample according to step ii.b.

In specific embodiments the invention therefore relates to a method for determining the presence of one or more target sequences in a polypeptide-comprising sample, comprising:

providing a nanopore sequencing library generated from the polypeptide-comprising sample,

subjecting the sequencing library to a nanopore sequencing process comprising the following analysis steps:

i. obtaining a polypeptide sequence by sequencing 10-1500 consecutive amino acids of a polypeptide fragment in a nanopore of a nanopore sequencing device,

ii. comparing the polypeptide sequence obtained in step i. to sequences comprised within a decoy sequence database and/or comparing the polypeptide sequence obtained in step i. to the one or more target sequences comprised within a target sequence database by applying a probabilistic data structure approach that utilizes hash functions, subsequences and bit arrays for sequence comparison,
wherein

a. if the sequence obtained in step i. matches to at least one decoy sequence comprised by the decoy sequence database but not to the sequences comprised by the target database, the sequencing of the respective polypeptide fragment is aborted and the polypeptide fragment is discarded from the nanopore, or

b. if the sequence obtained in step i. matches to at least one target sequence comprised by the target sequence database but not to the sequences comprised by the decoy sequence database the presence of the target sequence of the target sequence database with the highest shared sequence identity to the obtained sequence is determined, and preferably the sequencing of the polypeptide fragment is continued, preferably until the end of the polypeptide fragment is reached and a final sequence is obtained,
, or

c. if the sequence obtained in step i. neither matches to at least one sequence comprised by the decoy sequence database nor to at least one sequence comprised by the target sequence database, or

if the sequence obtained in step i. matches to at least one sequence comprised by both the decoy sequence database and the target sequence database,

the sequencing of the polypeptide fragment in the nanopore of step i. is continued for a defined length of amino acids, preferably at least 100 amino acids, or alternatively until the end of the fragment, resulting in an elongated sequence,

and the obtained elongated sequence is compared to sequences comprised within the decoy sequence database and/or to sequences comprised within the target sequence database according to step ii., wherein one of alternative steps ii. a., b. or c. is performed.

iii. optionally determining the presence of one or more target sequences in the sample according to step ii.b.

The invention further relates to a method for determining the presence of one or more target sequences in a nucleic acid-comprising sample, comprising:

providing a nanopore sequencing library generated from the nucleic acid-comprising sample,

subjecting the sequencing library to a nanopore sequencing process comprising the following analysis steps:

i. sequencing 10-1500 consecutive nucleotides of a nucleic acid fragment in a nanopore of a nanopore sequencing device,

ii. comparing the nucleotide sequence obtained in step i. to sequences comprised within a decoy sequence database and/or comparing the nucleotide sequence obtained in step i. to the one or more target sequences comprised within a target sequence database by applying a probabilistic data structure filter approach that utilizes hash functions, subsequences and bit arrays for sequence comparison, wherein

a. if the sequence obtained in step i. matches to at least one decoy sequence comprised by the decoy sequence database but not to the sequences comprised by the target database, the sequencing of the respective nucleic acid fragment is aborted and the nucleic acid fragment is discarded from the nanopore, or

b. if the sequence obtained in step i. matches to at least one target sequence comprised by the target sequence database but not to the sequences comprised by the decoy sequence database, preferably the sequencing of the nucleic acid fragment is continued, preferably until the end of the nucleic acid fragment is reached and a final sequence is obtained,
and determining the target sequence of the target sequence database with the highest shared sequence identity to the obtained sequence, or

c. if the sequence obtained in step i. neither matches to at least one sequence comprised by the decoy sequence database nor to at least one sequence comprised by the target sequence database, or

if the sequence obtained in step i. matches to at least one sequence comprised by both the decoy sequence database and the target sequence database,

the sequencing of the nucleic acid fragment in the nanopore of step i. is continued for a defined length of nucleotides, preferably at least 100 nucleotides,

and comparing the obtained elongated sequence to sequences comprised within the decoy sequence database and/or to sequences comprised within the target sequence database according to step ii., wherein one of alternative steps ii. a., b. or c. is performed,

iii. optionally determining the presence of one or more target sequences in the sample according to step ii.b.

It was entirely surprising that the present approach is able to reliably remove reads belonging to large reference sequences, while running on commodity hardware, e.g. a standard laptop or desktop computer. The present method drastically improves the potential enrichment of low abundance sequences by its high read classification sensitivity and specificity, which outperforms existing tools in the field.

As is shown in more detail below, the present approach enables, especially due to the new approach using a probabilistic data structure, the analysis of nanopore sequencing data using large reference genomes (e.g human genomes) or proteomes on regular computational means, such as laptops, and therefore enables even small sequencing facilities or in-field researchers that typically only have access to low-cost, compact and/or portable hardware to use the adaptive sampling feature of the MinION sequencer.

The present method also has the capacity to make a lasting contribution to establishing nanopore sequencing as a point-of-care test for pathogen detection in small hospitals or medical practices, where it is crucial to enrich for low abundance pathogen nucleic acids or polypeptides in a patient sample.

In embodiments of the invention the probabilistic data structure is selected from the group comprising an interleaved bloom filter (IBF), an interleaved XOR filter or an interleaved binary fuse filter.

In specific embodiments the biopolymer molecules that are rejected in the present method, e.g., according to step ii.a, are on average about 100 building blocks, e.g. nucleotides or amino acids, shorter than those from the comparable state-of-the-art method Readfish. Hence, the sequencing at a nanopore can be aborted earlier than in prior art methods, if a sequence that is not of interest or considered background or contamination is present within a nanopore, which has a significant beneficial impact on analysis time, efficiency and throughput.

Surprisingly in this specific embodiment the present method enables thus the translation of said about 100 building blocks, e.g. nucleotides or amino acids, into about 0.2 seconds less time spent sequencing an uninteresting biopolymer, e.g. nucleic acid, molecule when compared to other state-of-the-art methods. This specific embodiment provides a non-limiting example of how the present method can improve analysis speed, throughput and efficiency, amongst others, by requiring shorter biopolymer, e.g. nucleic acid, sequences for rejection and/or classification decisions, which translates into a shorter and improved total analysis time and a faster detection of sequences of interest. This effect is enabled by the use of a probabilistic data structure approach in the present method. Therein the comparison of short, sequenced fragments to sequences comprised within reference database(s) (decoy and/or target databases) can be performed in preferred embodiments without classical sequence alignment but only by using the present probabilistic data structure approach.

In embodiments, the in step i. Nanopore-sequenced biopolymer fragment or biopolymer sequence may be between 10-1500 consecutive building blocks. In some embodiments this sequence may be a specific short barcode sequence that was ligated to a biopolymer fragment, e.g. during a barcoding step while sequencing library preparation.

In other embodiments, the in step i. Nanopore-sequenced biopolymer fragment or sequence is between 50-1500 or between 100-1500 building blocks in length, enabling the identification and/or assignment of a specific or characteristic biopolymer sequence to a target and/or decoy sequence comprised within the target and/or decoy database.

Classical alignment approaches suffer from high computing and memory requirements and thus are not suitable for adaptive sampling. They simply could not classify the sequenced reads in real-time applications. Current state-of-the-art adaptive sampling approaches rely on long-read mapping software, that uses heuristics and FM-Index data structures to align the sequenced reads to references sequences in real-time. However, they suffer from high memory requirements when the target or decoy index consist of more than one human-sized reference genome or proteome. They also do not report alignments to repetitive regions in the reference genome or proteome since it cannot equivocally decided at which position the read matches best. Although, so far probabilistic data structures, such as interleaved Bloom filters, have been applied in nucleic acid sequence analysis software, they were only utilized for high accuracy short read sequencing technologies, such as Illumina, where higher, more specific k-mer values can easily be used. In addition, they have never been applied to real-time sequencing applications, especially for long-read nanopore sequencing because the high error rates of the technology seemed to make the not applicable.

Surprisingly, with the methods according to the invention, the usage of short subsequences, e.g. k-mers, for real-time read classification of error-prone nanopore reads can now be established using the probabilistic data structure and achieving sufficient sensitivity and specificity. Classical sequence alignment methods require longer sequence fragments, compared to the present approach, and extensive computational resources with high processing and storage capabilities, such as a GPU, instead of a CPU. Hence, the present method provides the improvement over the prior art that it can perform sequence analysis in shorter time and on standard laptops or desktop computers without the need for computational expert knowledge or powerful, large and expensive computing devices. This reduction in sequencing time can decrease the time to answer a biological question and can increase, through the improvement of nanopore adaptive sampling by the present probabilistic data structure approach, the enrichment of low abundant but interesting biopolymer sequences compared to other tools.

A further surprising key benefit of the present method is the improved read classification. In contrast to other nanopore adaptive sampling tools, the present method neither uses signal nor sequence space mapping algorithms for read classification. Instead, the present approach uses subsequences, such as k-mers, minimizers or strobemers, and probabilistic data structures, such as Bloom filters, XOR filters or binary fuse filters, for sequence containment testing, resulting in smaller index files and fewer computer memory requirements. This also results in a significantly higher sensitivity compared to prior art sequence analysis tools used for read-classification in adaptive sampling, while having the same specificity as the best competitor method Readfish.

In one aspect, the present invention relates to a new nanopore sequencing approach comprising nanopore adaptive sampling that combines fast base calling with read classification based on probabilistic data structures, such as Interleaved Bloom filters (IBF), interleaved XOR filters or an interleaved binary fuse filter. In embodiments the present method surprising robustly removes even reads belonging to large reference sequences while running on commodity hardware, preferably using a CPU instead of a graphical processing unit (GPU). One of its surprising benefits is that the present method drastically improves the potential enrichment of low abundance sequences by its high read classification sensitivity and specificity, which outperforms existing tools in the field. Prior art methods are based on dynamic time warping algorithms that need GPUs for signal alignment and cannot handle human-sized reference sequences. Other prior art methods apply read mapping in sequence space but rely on fast GPU base callers for real-time read rejection. Using nanopore long-read mapping tools is also not optimal when trying to map shorter reads. The present method revolutionizes adaptive sampling by overcoming these issues and making it accessible for in-field researchers. It also provides a user-friendly interface for end-users without a bioinformatics background.

In embodiments of the present invention, the comparing and matching of step (ii) does not comprise or involve sequence alignment.

In a preferred embodiment the invention relates to a method for determining the presence of one or more target sequences in a biopolymer-comprising sample, wherein the decoy and/or the target sequence database are dynamically indexable databases, which can be modified during any of the method steps i. to iii, wherein the decoy and/or the target sequence database are preferably modified according to the target sequences determined under step ii.

One advantage of dynamically indexable databases is that reference sequences can be added, adapted or removed from the database during the sequencing and/or analysis run. Hence, the reference database can be modified, for example, depending on the target organisms or target biopolymer detected during the sequencing run. This enables, in one embodiment, during the sequencing run the addition or removal of more specific sequence information on members of a certain target organism or pathogen family that is detected, or on specific mutations leading to substance sensitivity or resistance of a certain pathogen. For example, in one embodiment not every genotype of a pathogen family needs to be comprised by the initial database, as upon detection of a certain pathogen family or species, based on a representative reference sequence, more detailed information on every genotype comprised within said family may be added during the sequencing run and/or analysis. A genotype of interest may be a virulent or pathogenic strain or variant of a pathogen and/or a specific mutation leading to the resistance to a anti-microbial, virostatic and/or antibiotic substance. Genotypes or specific mutations in a pathogen leading to the sensitivity to a certain medicament, antibiotic or virostatic substance may also be of interest. In the context of the present invention any genotype, mutation or gene-variant may be of interest. Accordingly, a mutation leading to an adaptation to an environmental condition or relating to a certain strain or variant of a (micro)organism may also be of interest in an environmental sample. Accordingly, due to this fluent adaptation of reference data, based on the actually detected targets (e.g. pathogens), the initial reference/target database only needs to comprise biopolymer sequence information representative of, for example, (micro)organism families, species or strains, resulting in a smaller initial database size initially and throughout the sequencing process. For example, depending on the initially detected target sequences, the target database can be updated by including more specific sequences that are related to the detected target sequence, while non-detected sequences that were initially comprised by the target database can be removed. On the other hand, in embodiments it is also possible to modify the decoy or target databases depending on the reference sequence coverage. For example, in embodiments, if a certain sequencing coverage of a sequence comprised in the target database has been reached, this reference sequence is moved to the decoy database because there are no more sequences needed for this reference. Further, in embodiments it is also possible to choose the correct reference sequences of the decoy database depending on the sequencing information from the first couple of minutes of a sequencing run, choosing or suggesting for instance the best matching host reference. In favor of a small target database, in embodiments it is also possible to choose target sequences based on symptom information of the patient and choose only reasonable pathogen sequences as targets. In the context of embodiments of the present invention a smaller reference database also has the beneficial effect of less storage capacity required on the local computing device and a faster comparison of the obtained sequences to the target and/or decoy database.

In one embodiment the invention relates to a method for determining the presence of one or more target sequences in a biopolymer-comprising sample, wherein if the obtained and/or final sequence matches to at least one sequence comprised by both the decoy sequence database and the target sequence database, the target sequence comprised by the target sequence database with the highest shared sequence identity to the obtained and/or final sequence is determined and optionally the presence of one or more target sequences in the sample is determined according to step iii.

In a preferred embodiment of the invention the comparison in step ii. comprises applying a 95%-confidence interval (CI) for the number of matching subsequences, such as, e.g. k-mers, between the biopolymer fragment sequenced in step i. and a sequence from the decoy and/or the target sequence database. In other embodiments statistical inference is performed and/or confidence intervals are constructed for the quantity of organisms. Statistical inference generally refers to the process of using data analysis to infer properties of an underlying distribution of probability. Inferential statistical analysis infers properties of a population, for example by testing hypotheses and deriving estimates. It is assumed that the observed data set is sampled from a larger population.

In another embodiment of the invention the comparison in step ii. comprises applying a 70%, 75%, 80%, 85%, 90%, 91% 92%, 93%, 94%, 96%, 97%, 98%, 99%,99.5% or 99.9%-confidence interval for the number of matching subsequences, such as k-mers, between the biopolymer fragment sequenced in step i. and a sequence from the decoy and/or the target sequence database.

In one embodiment the present method may further apply an expected sequencing error rate of 1%, 2%, 3%, 4%, 5%, 6%, 7%, 8%, 9%,10%, 15%, 20%, 25% or 30% and k-mer length of 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 45, 46, 47, 48, 49, 50, 51, 52, 53, 54, 55, 56, 57, 58, 59 or 60. However, these values are adjustable via configuration parameters of the command line or graphical user interface (GUI) and depend, among others, on the sequencing accuracy provided by the current nanopore sequencing device that is used.

In a preferred embodiment the sequencing error rate is set according to the current nanopore sequencing error rate of the device used with the present method, such as the MiniOn device, which is currently 10%, hence the error rate is set to 10% and the k-mer length is set accordingly to 13. Improvements on the sequencing error rate of nanopore devices in the future can result in preferred error rates below 10% and sizes of subsequences, such as k-mers, above 13. Recent advancements in basecalling algorithms facilitated on GPUs show error rates of 7% and smaller, for which subsequence (e.g. k-mer) lengths of 15 or 17 are, in embodiments, feasible as well. In embodiments longer subsequences mean that longer reference fragment sizes can be used for the IBF approach, which increases the performance (speed) of the sequenced fragment classification.

In general, a lower error rate in sequencing has the positive effect that greater lengths of subsequences, e.g. k-mers, can be used without negatively affecting the sensitivity / specificity of the classification. In embodiments of the present invention the use of larger subsequences, e.g. k-mers, can once again reduce the memory requirements for indexable databases and increase the speed of the classification enormously.

In a further embodiment of the method of the invention, if the sequence obtained in step i. neither matches to a sequence comprised by the decoy sequence database nor to a sequence comprised by the target sequence database, the comparing step ii. is repeated, preferably at least one time, before the sequencing of the biopolymer fragment in the nanopore is continued according to step ii. c. In one embodiment this means that based on the classification, reads can either be marked for rejection from a nanopore according to step ii.a, or sequencing is continued for said biopolymer fragment in the nanopore of the sequencer. In one embodiment, if a read was not classified for rejection on a first attempt, in can be marked as "analyzed once" and the sequencing is continued and the additionally acquired sequencing data is subjected to another classification attempt of that read/ biopolymer sequence. In embodiments, after the read could not be classified for rejection for at least one time, at least two times, at least three times, at least four times, at least five times, at least six times, at least seven times, at least eight times, at least nine times, at least ten times it is marked for continued sequencing as usual or for sequencing until the end of the fragment. In further embodiments, after the read could not be classified for rejection for a first time, a second time, a third time, a fourth time, a fifth time, a sixth time, a seventh time, an eighth time, a nineth time or for ten times it is marked for continued sequencing as usual or for sequencing until the end of the fragment.

One of the benefits of embodiments in which if the sequence obtained in step i. neither matches to a sequence comprised by the decoy sequence database nor to a sequence comprised by the target sequence database, the comparing step ii. is repeated, preferably at least one time, before the sequencing of the biopolymer fragment in the nanopore is continued according to step ii. c., is that it saves analysis time, as the in-silico comparison can be repeated faster than additional data from sequencing can be obtained. This re-analyzing comprises in one embodiment the comparison of the obtained biopolymer sequence with the probabilistic data structure approach. If no match can be found, the comparison can be repeated again, until a predetermined number of comparisons (re-analyzing rounds) has been performed and sequencing is continued, or until a match can be determined. This can facilitate a faster decision, if the sequence is not or is indeed comprised within the decoy database and/or the target database. Accordingly, the duration of the analysis can be optimized, as a sequence that should be rejected, but was not matched in the first attempt, has another chance of being matched and rejected before it is further sequenced. In this case an uninteresting (e.g. host/decoy/contamination) sequence can be rejected faster and the nanopore is vacated for the next biopolymer fragment to attach. Consequently, in this specific embodiment analysis performance and speed is also increased if a sequence can be quickly matched to a target sequence through repeated comparisons with the IBF-approach, as the decision to continue sequencing can also be made faster, which avoids repeated sequencing of short fragments and subsequent matching to the sequences within the databases. In addition, in embodiments of the present method this can also lead to a reduction of costs. Further, as the performance of the pores declines over time, even the quality of the results may potentially be preserved on a high level.

In addition, the advantages of embodiments of the invention are not only limited to the reduction of analysis time, costs and the improvement of analysis quality, but also allow increasing the dynamic range and detection threshold of the technique. In embodiments the selective sequencing facilitates to detect targets of an order of magnitude lower concentration as possible with prior art methods - as embodiments of the invention can be considered an (*in silico*) enrichment approach. Also, embodiments of the invention can be regarded as a quality control procedure (an analysis run can be aborted if not enough of the desired target sequence is found after a certain time, or a subsequent in-depth analysis can be started on another sequencing machine, e.g. Illumina sequencing analysis).

Further, in embodiments there are also advantages for privacy preserving aspects, e.g. when a protocol for filtering out human sequences, such as proposed in Loka et al. (Bioinformatics, Volume 34, Issue 14, 15 July 2018, Pages 2376-2383), is applied, before data is obtained. Thereby in embodiments privacy by design standards of GDPR can be ensured. This aspect is a considerable advantage of embodiments of the present approach because a human background can be added, while ensuring that there is not enough human biopolymer information, e.g. nucleic acid or polypeptide information, to reliably (re-)identify a unique human. Also, in embodiments another way performing the present approach, sequence coverages can be balanced across different genomic regions, which enables subsequent bioinformatics/statistical approaches, which intrinsically rely on a certain assumption.

In one embodiment, the method of the invention may be considered a computer implemented method. In embodiments of the computer implemented method sequence reads that have been classified for rejection or for continued sequencing are finally pushed/send to a "response queue". The last thread (process step) takes the classified reads from the response queue, and a "Read-Until" client sends response messages back to the control software of the nanopore sequencing device (e.g., MinKNOW) for each read. The client sends an unblock message for sequence reads that could be matched to the probabilistic data structure, e.g. in specific embodiments an Interleaved Bloom filter (IBF), of the decoy database, telling the sequencer to eject the corresponding biopolymer molecule from the nanopore. In other words, in embodiments the last step of analysis is the communication between the Nanopore sequencer und the present method regarding whether the respective biopolymer fragment in a pore should be ejected from the pore or not. In embodiments this leads to the voltage that is applied to the pore being reversed, such that the biopolymer fragment e.g. a negatively charged nucleic acid fragment, is pushed backwards out of the pore. Hence, if a biopolymer fragment only matches to one or more sequences of the decoy database and not to one or more sequences of the target database, in embodiments a message is send back to the nanopore sequencer, causing the ejection of the aforementioned biopolymer fragment from the nanopore.

In one embodiment of the computer implemented method a "stop further data"-message is sent to the control software of the nanopore sequencer for reads that were not classified for rejection. This message tells the control software of the nanopore sequencer (e.g., MinKNOW) to continue sequencing of the corresponding biopolymer molecule in the nanopore and send no additional chunks of data to the analysis computer for that biopolymer sequence, but to obtain a final sequence of the entire biopolymer molecule. In other words, if a biopolymer fragment matches to one or more sequences of the target database, in embodiments a message is send back to the nanopore sequencer, causing it to continue sequencing until the end of aforementioned biopolymer fragment. This also causes the sequencer to avoid sending further sequencing data for that fragment for the real-time classification described in ii a) to c). Hence, in embodiments only a final sequence of the entire biopolymer molecule is obtained at the end.

In a further embodiment the invention relates to a method for determining the presence of one or more target sequences in a biopolymer -comprising sample, wherein if the obtained and/or final sequence matches to at least one sequence comprised by both the decoy sequence database and the target sequence database, the target sequence comprised by the target sequence database with the highest shared sequence identity to the obtained and/or final sequence is determined and optionally the presence of one or more target sequences in the sample is determined according to step iii. This embodiment has the advantage that biopolymer sequences that could be matched to both the decoy database and the target database are not excluded from the sequencing analysis and/or the final results and subsequent downstream analysis. This has the advantage that such sequences are not "lost" but completely characterized by sequencing and can be analyzed or discarded at a later data evaluation step. This approach, for example, has a great advantage especially when the sequencing error rate is high and/or closely related organisms or sequences are comprised within the decoy and the target database.

In a further embodiment the invention relates to a method for determining the presence of one or more target sequences in a biopolymer-comprising sample, wherein the nanopore sequencing analysis is continued until a preset number of biopolymer fragments have been analyzed by steps i. and ii, or until a preset total analysis time has been reached.

The duration and/or extend (maximum number of biopolymer fragments to be analyzed) of performing the method according to the invention can be preset according to the desired depth of information to be acquired, the amount of starting material used, the number of nanopores available for the analysis, the expected sequencing error, the expected concentration of the target and/or decoy biopolymer sequences within the sample, or the time or reagents available for the analysis method according to the invention.

In another embodiment the present method can also be aborted or extended beyond a present limit, for example according to the information acquired during the sequencing run or other parameters. Namely, the preset analysis time or maximum number of biopolymer sequences to be analyzed can be changed or modified during the sequencing and/or analysis process, for example, according to sequences detected during the sequencing run. This flexibility has the advantage that, if, for example, unexpected and/or highly interesting sequence information is detected during the sequencing and/or analysis, that a preset analysis time or a preset maximum number of fragments to be analyzed can be modified, such that the depth of analysis and the amount of acquired information can be, e.g. extended or increased. In other situations, the method according to the invention can be aborted before the preset parameters are reached.

The entire sequencing run can be terminated anytime by the user through the command line or the graphical user interface, or a maximum total analysis time, or a maximum number of biopolymer fragments that have to be analyzed can be preset.

In one embodiment the maximum total analysis time may be set to 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 45, 46, 47, 48, 49, 50, 51, 52, 53, 54, 55, 56, 57, 58, 59 or 60 minutes, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 45, 46, 47, 48, 49, 50, 51, 52, 53, 54, 55, 56, 57, 58, 59 or 60 hours.

In one embodiment the maximum total number of biopolymer fragments to be sequenced and/or analyzed according to the invention may depend on the nanopore device used and the available number of nanopores within the device, on the sequencing reagents, on the computing device and/or on the concentration of the target and/or decoy biopolymer in the sample. In one embodiment the maximum number of biopolymer fragments to be analyzed may be set to 1000, 5000, 10,000, 20,000, 30,000, 50,000, 100,000, 150,000, 250,000, 500,000, 1 million, 1,5 million, 2 million, 5 million, 10 million, 25 million, 50 million or even 100 million biopolymer fragments.

In a preferred embodiment the invention relates to a method for determining the presence of one or more target sequences in a biopolymer-comprising sample, wherein the one or more target sequences comprise sequences comprised by a pathogen (pathogen sequence), wherein the pathogen is preferably selected from the group of bacteria, viruses, fungi, protists, parasites and/or plasmodia.

A sample herein may comprise multiple different pathogens or microorganisms, wherein some may be considered pathogenic or of interest (target), while others may be comprised within the decoy database as they are not of interest. For example, in a patient sample the biopolymer information of a certain strain or genotype of bacteria, which comprises a resistance gene to a certain substance (e.g., an antibiotic), may be of interest and comprised within the target database. In the same case bacteria from the same family might be sensitive to a certain substance, as it lacks the resistance gene of interest, and its biopolymer information might hence be comprised within the decoy database. In the case of a patient sample, for example from a human or an animal subject, the decoy database might also comprise the biopolymer sequences of the "host", namely the patient the sample was taken from. Such patient decoy biopolymer sequences might be sequences generally representing the human genome or proteome or the genome or proteome of said animal, e.g., *mus musculus,* or might represent the individual genome or proteome of said patient.

The same complexity might apply to embodiments relating to agricultural or environmental samples, e.g., soil or water samples, where the biopolymer sequence information of certain strains or genotypes of microorganisms are comprised within the target database and those of other microorganisms might be comprised within the decoy database.

In the context of the present invention, it is further possible to use the invention for pathogen detection in wildlife and domesticated animals. In such cases, the entire reference genome or proteome of the host organism is not always available. In this case the present invention facilitates in some embodiments the use of an available reference sequence of a closely related species to deplete the host reads during sequencing. This possibility provides a high flexibility for the application of the present invention and enables the application even in field research. An example of this specific embodiment can be found in the following examples, where nanopore reads from the *rattus norvegicus* were successfully queried against the reference genome of *mus musculus.*

In other embodiments the decoy database may comprise the biopolymer information acquired from an earlier reference sample, e.g. an environmental reference sample or veterinary reference sample, that was taken, for example, at another sampling site and/or at another time. Hence, the present method could in such embodiments serve to identify changes in organisms or genotypes present in the sample compared to earlier time points or different sites of sampling.

In other embodiments a sample may also comprise contaminants, for which corresponding reference sequences might be added either to the decoy or the target database, depending on the biological question to be answered.

In a further embodiment the invention relates to a method for determining the presence of one or more target sequences in a biopolymer-comprising sample, wherein the target sequence database comprises one or more pathogen sequences comprising a mutation, a genotype of interest, a resistance, and/or a sensitivity to a certain substance.

This might in some embodiments be the sensitivity/susceptibility or resistance to a certain medicament, antibiotic or virostatic. Such sensitivity/susceptibility or resistance might in some cases be caused by genetic mutations or inherent to specific genotypes or strains of an organism.

As the present method enables an improved analysis of nanopore sequencing data of entire genomes that is faster and more computer-memory efficient than prior art methods, it qualifies especially for point-of-care use and in agriculture. Especially in these fields it can be of high interest to detect mutations or genotypes that are associated with substance resistance or sensitivity, or certain beneficial or malignant characteristics, such as antibiotics-resistance mutations or pathogen subtypes. In these contexts, it is of importance that pathogens with certain genotypes can be detected fast und without special computational equipment, enabling a fast and easy analysis with high sensitivity on the site of sampling.

In a further embodiment the invention relates to a method for determining the presence of one or more target sequences in a biopolymer -comprising sample, wherein the decoy sequence database comprises one or more biopolymer sequences representative of a host species, such as genomic nucleic acid sequences or corresponding polypeptide sequences, mitochondrial nucleic acid sequences or corresponding polypeptide sequences, transient nucleic acid sequences or corresponding polypeptide sequences, vector nucleic acid sequences or corresponding polypeptide sequences, or any combination thereof.

In a further embodiment the invention relates to a method for determining the presence of one or more target sequences in a biopolymer-comprising sample, wherein the host species is selected from the group of bacterial, viral, fungal, protist, plasmodia, eucaryotic, amphibian, avian, plants, preferably mammal or human.

In a further embodiment the invention relates to a method for determining the presence of one or more target sequences in a biopolymer-comprising sample, wherein the biopolymer-comprising sample is a sample derived from a subject, a patient, a cell culture of patient cells or cell lines, an animal, or a cell culture of animal cells or cell lines, a biopsy, a blood sample, a tissue sample, an environmental sample, or a food-derived sample.

One of the great advantages of the present method is that it is especially useful in the sequencing of metagenomics samples, where enrichment of low-abundance genomes can reduce the time and costs required to answer a biological question (time-to-answer) and improve the ability to assemble these low copy genomes. The same applies to samples with low abundancy of target proteomes. This is especially interesting for pathogen detection in human or animal samples that suffer from up to 99.99% of sequenced reads being host background.

Other examples for the application of the present method are the enrichment of known tumor specific sequences by depleting all other human genes, or the enrichment of human exons by depleting intron sequences in a human sample.

In a preferred embodiment the invention relates to a method for determining the presence of one or more target sequences in a biopolymer-comprising sample, wherein the biopolymer is a nucleic acid, selected from the group comprising DNA, gDNA, cfDNA, RNA, gRNA, mRNA and cDNA, or any combination thereof.

In a preferred embodiment the invention relates to a method for determining the presence of one or more target sequences in a biopolymer-comprising sample, wherein the biopolymer comprises or is one or more or a plurality of polypeptides and/or proteins or any combination thereof.

In a further embodiment the invention relates to a method for determining the presence of one or more target sequences in a biopolymer-comprising sample, wherein the sequencing in step i. comprises base calling step, which is achieved by a computational base calling algorithm executed on a Central Processing Unit (CPU), a Graphics Processing Unit (GPU), a Tensor Processing Unit (TPU) or a Field Programmable Gate Array (FPGA) of a computer connected to or comprised within said nanopore sequencing device.

The present method might execute the base calling algorithm on any of the before mentioned units, although the capability of the present invention to execute the base calling even on a CPU has great advantages over the prior art methods, that usually require a GPU for this task. This capability facilitates the use of standard computational equipment, such as a standard laptop, for sequence analysis, without the need for computational equipment with large computation capacity or a GPU. Hence, the present CPU-based approach provides access to adaptive sampling much easier, for example for researchers studying wild living animals in the field or to doctors at the point-of-care, as even sequencing reads belonging to large reference sequences can be analyzed, using commodity hardware without a GPU.

One of the features of the invention enabling this highly storage and processing capacity efficient sequencing analysis is the use of the present probabilistic data structure approach in the context of the present method, instead of classical prior art base calling and sequence alignment methods, which require extensive storage and processing capacity.

In contrast to classical alignment methods, in embodiments of the present approach biopolymer sequences are not compared directly by alignment when using the IBF approach. In embodiments of the present invention the sequences are subdivided into subsequences of length k, for which a numerical value is computed by using hash functions. Based on that value, single bits are set in a bitvector indicating the presence of the corresponding k-mer within the biopolymer sequence. Storing only the bitvectors results in less memory requirements and allows the utilization of fast parallel bitvector operations for sequence comparison purposes. In embodiments the present approach also allows the parallel comparison of small/short biopolymer fragments with databases comprising a large amount of reference sequences.

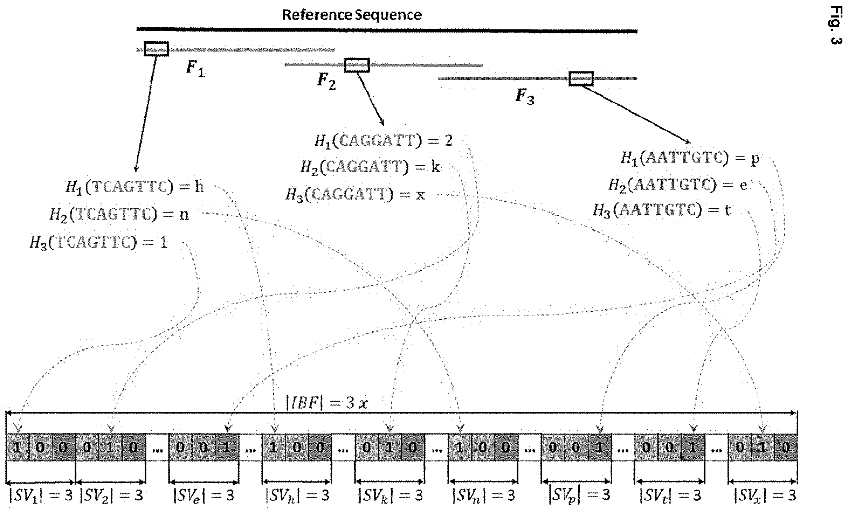

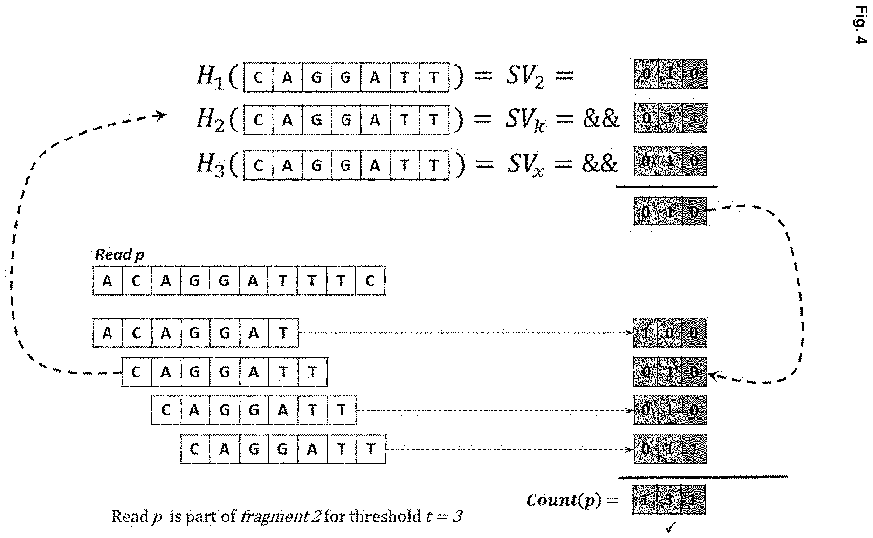

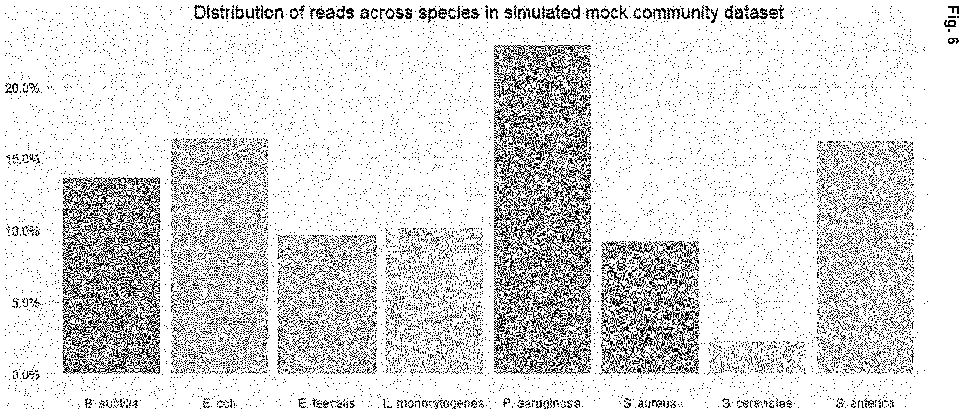

In a preferred embodiment the invention relates to a method for determining the presence of one or more target sequences in a biopolymer-comprising sample, wherein steps i. to ii. are performed in parallel in multiple nanopores of the nanopore sequencing device In one embodiment of the present invention, which is also depicted in Figures 3 and 4, the probabilistic data structure, which is in this specific embodiment an interleaved Bloom filter (IBF), is constructed as follows (detailed explanations of the specific terms used in the following can be found in the detailed description): In the first step, the reference sequence is subdivided into three overlapping fragments. Then, for each k-mer of the differently colored fragments, all three hash values have to be calculated. The resulting hash values determine the subvector SV3 in which the corresponding bit is set to 1. For example, the second hash function for k-mer AATTGTC from fragment F3 returns e. Hence, the third bit of subvector SVe was set to 1. In this way, the three Bloom filters for the three fragments are combined in an interleaved fashion. Since there are three fragments in this example, the length of every subvector is three, and the length of the IBF is 3x, where x is the defined length for every Bloom filter of the three fragments. Finding the correct fragment for a given read p. For each k-mer of read p, the three hash values are calculated using the same hash functions as for the IBF construction. The resulting hash values are used to find the corresponding subvectors of the IBF. The sub bitvectors are combined with a bitwise AND to a binning bitvector. For all set bits in the binning vectors of the subsequences, which are in this embodiment k-mers, the counter of the corresponding bin are incremented in a counting vector. Bins whose counter is greater than or equal to a given threshold t are considered to contain the read p. In this example, the calculation of the binning bitvector for the 7-mer CAGGATT is shown. Using the same three hash functions as for the IBF construction in Figure 6, the subvectors $SV2$, $SVk$, and $SVx$ are obtained. These three subvectors are combined via logical AND to get the binning bitvector. The same procedure is applied to the other three 7-mers, and with the resulting four binning bitvectors, the number of matching 7-mers of read p can be calculated with each fragment. If at least three 7-mers match against one fragment, the read is accepted as a match with the reference sequence.

In embodiments, as a first step, the present method produces overlapping fragments of each of the given reference sequences comprised within the target or decoy database with a defined overlap of 1000 base pairs, wherein each of those fragments represents a single bin in the probabilistic data structure, which is in the present non-limiting example an Interleaved Bloom filter. The constituting k-mers of each fragment are hashed using three different hash functions, and the bits of the corresponding index positions in the probabilistic data structure, here an Interleaved Bloom filter (IBF), are set to one. Then, the present method automatically calculates the optimal IBF size in bits (Bits$_{IBF}$) based on the following equations.

$$Bits_{IBF} = n_{frag} \times Bits_{SBF}$$

$$[\ Bits_{IBF} = n_{frag} \times Bits_{SBF}\ ]$$

Where frag is defined as the number of fragments with maximum size F and Bits$_{SBF}$ as a single Bloom filter size for a single fragment. Let $max_{kmer}$ be the maximum number of k-mers for a fragment of size F, and k-mer size k be defined as

$$max_{kmer} = F - k + 1$$

$$[\ max_{kmer} = F - k + 1\ ]$$

To calculate the optimal size for the IBF, the inventors used the formula for finding the false positive rate in an IBF as proposed by Dadi et al. 2018.

$$p = \left(1 - \left(1 - \frac{1}{Bits_{SBF}}\right)^{h \times max_{kmer}}\right)^{h}$$

$$[\quad p = (1 - (1 - 1/Bits_{SBF})^{h \times max_{kmer}})^{h} \quad]$$

Then the optimal size of a single Bloom filter can be calculated by resolving the formula for $Bits_{SBF}$:

$$Bits_{SBF} = \left\lceil \frac{-1}{(1-r)^{\frac{1}{h \times max_{kmer}}} - 1} \right\rceil$$

$$[\quad Bits_{SBF} = [-1 \,/\, (1-r)^{\,1/(h \times max_{kmer})-1}] \quad]$$

Where $r = p^{\frac{1}{h}}$, $h$ is the number of used hash functions and p a predefined false positive rate. The present method implicitly uses three hash functions and a maximum false positive rate of 0.01 to minimize the number of false matches between the query sequence and a single bin of the probabilistic data structure, that is in the present example an Interleaved Bloom filter. In further embodiments other parameters may be applicable. In embodiments where a reduction of the size of the IBF (or the database) is desired, the maximum false-positive rate may be set to, e.g., 0.02, 0.03, 0.04 or 0.05. In embodiments it is further possible to increase the number of hash functions to achieve greater sensitivity or to reduce the database size (here, e.g., 2, 4 or 5 hash functions could be applicable).

During the classification step, the k-mers of every currently sequenced biopolymerfragment are hashed with the same three hash functions, and the number of matching k-mers for every bin is calculated as visualized in Figure 4. A biopolymer fragment is accepted as part of a sequence comprised in one of the databases if the number of matching k-mers is greater than or equal to a given threshold t for at least one bin. The threshold is calculated using the expected sequencing error rate e and the definition of a *(1 - a)* confidence interval of the number of erroneous k-mers, wherein the expected number of erroneous k-mers is calculated as follows:

$$E[N_{err}] = L \times q$$

For a given biopolymer fragment r with length *len(r)* and k-mer length *k,* the number of k-mers of fragment *r* is denoted as $L = len(r) - k + 1$, and *q* is defined by $(1 - (1 - e)^k)$. In a second step the variance for the number of erroneous k-mers can be calculated by

$$Var(N_{err}) = L(1-q)\left(q\left(2k + \frac{2}{e} - 1\right) - 2k\right) + k(k-1)(1-q)^2 + \frac{2(1-q)}{e^2}((1 + (k-1)(1-q))e - q) \quad (10)$$

Finally, the (1 - *a*) confidence interval is defined by:

$$E[N_{err}] \pm z_\alpha \sqrt{Var(N_{err})}$$

With $z_\alpha = \varphi^{-1}(1 - a/2)$, where $\varphi^{-1}$ is denoted as the inverse of the cumulative distribution function of the standard Gaussian distribution. Based on the calculation of the confidence interval for the number of erroneous k-mers, the inventors defined the threshold for the minimum number of matching k-mers for biopolymer fragment *r* as:

$$min[N_{match}] = L - \left( E[N_{err}] + z_\alpha \sqrt{Var(N_{err})} \right)$$

A biopolymer fragment is classified as a match if the number of matching k-mers is bigger or equal to *min*[$N_{match}$] for at least one bin in the probabilistic data structure, e.g. here IBF. In one embodiment the present method calculates this threshold for a 95%-confidence-interval, an expected sequencing error rate of 10%, and k-mer length 13. However, these values vary depending on the accuracy of the sequencing reaction, *other parameters* set for the present method and the specificity und sensitivity desired for the present approach. The parameters can be different in other embodiments and are in the computer implemented method and the computer program adjustable via configuration parameters of the command line or a graphical user interface (GUI).

In another embodiment, the length of the subsequences, such as e.g. k-mers, may be 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 45, 46, 47, 48, 49, 50, 75, 100, 150, 200.

In another embodiment, the confidence interval may be 50%, 55%, 60%, 65%, 70%, 75%, 80%, 85%, 90%, 91%, 92%, 94%, 96%, 97%, 98%, 99%, 99,5%, 99,9%, 100%. In a preferred embodiment the confidence interval is 95%.

In another embodiment, the expected sequencing error rate is 0,01%, 0,05%, 0,1%, 0,5%, 1%, 1,5%, 2%, 2,5%, 3%, 3,5%, 4%, 4,5%, 5%, 5,5%, 6%, 6,5%, 7%, 7,5%, 8%, 8,5%, 9%, 9,5%, 10%, 11%, 12%, 13%, 14%, 15%, 16%, 17%, 18%, 19%, 20% or even 25%. Sequencing error rates above 20% are not advantageous or desired in the context of the present invention, as higher error rates would likely impede confident assumptions and reliable data evaluation.

The invention further relates to a device or system comprising means for performing nanopore sequencing and additional means for performing the method according to the invention.

In a preferred embodiment the invention relates to a device or system comprising means for performing nanopore sequencing and additional means for performing the method according to the invention, the device or system further comprising at least one computer-readable storage medium having stored thereon a computer program comprising instructions to cause the device or system to execute the steps of the method according to the invention.

In a further embodiment the invention relates to a to a device or system comprising means for performing nanopore sequencing and additional means for performing the method according to the invention, wherein the device or system is preferably a point-of-care (POC) device or system.

The use of embodiments of the present invention is particularly beneficial in a POC setting, since the present approach can be performed on standard computer equipment in combination with lab-free sample preparation methods allowing fully remote operations, without the access to labs or high-tech computers.

In a further embodiment the invention relates to a to a device or system comprising means for performing nanopore sequencing and additional means for primer forming the method according to the invention, the device or system further comprising means for isolating biopolymers, e.g., nucleic acids and/or polypeptides/proteins, from a sample and/or means for preparing a nanopore sequencing library.

The invention further relates to a computer program [product] comprising instructions which, when executed by a device or system according to the invention, cause the device or system to carry out steps i. to ii, and optional step iii. of the method according to the invention, and

optionally subsequently causes the device or system to save a final output summary comprising the sequencing information determined in each succession of steps i. to ii, and optional step iii. on a computer-readable storage medium,

wherein, while carrying out steps i. to ii, and optional step iii. of the method, the computer program interacts with the nanopore sequencing device or system to control the sequencing and discarding of the biopolymer at the individual nanopores of the nanopore sequencing device.

The invention further relates to a computer-readable medium having stored thereon the computer program according to the invention.

The invention further relates to a computer-implemented method for determining the presence of one or more target sequences in a biopolymer-comprising sample, comprising:

i. receiving sequencing data representing the initial 10-1500 consecutive building blocks of a biopolymer fragment acquired by a nanopore sequencing device during an ongoing nanopore sequencing process,
ii. comparing the biopolymer sequence received in step i. to sequences comprised within a decoy sequence database and/or comparing the biopolymersequence received in step i. to the one or more target sequences comprised within a target sequence database, wherein

a. if the sequence received in step i. matches to at least one decoy sequence comprised by the decoy sequence database but not to the sequences comprised by the target sequence database, instructions are sent to the nanopore sequencing device to abort the sequencing of the respective biopolymer fragment and to discard the biopolymer fragment from the nanopore of the nanopore sequencing device, or

b. if the sequence obtained in step i. matches to at least one target sequence comprised by the target sequence database but not to the sequences comprised by the decoy sequence database, preferably instructions are sent to the nanopore sequencing device to continue the sequencing of the biopolymer fragment in the nanopore of the nanopore sequencing device preferably until the end of the biopolymer fragment is reached and a final sequence is obtained, and

receiving sequencing data from the nanopore sequencing device representing the obtained sequence of the biopolymer fragment, and

determining the target sequence of the target sequence database with the highest shared sequence identity to the obtained sequence,

c. if the sequence received in step i. neither matches to at least one sequence comprised by the decoy sequence database nor to at least one sequence comprised by the target sequence database, or

if the sequence obtained in step i. matches to at least one sequence comprised by both the decoy sequence database and the target sequence database,

instructions are sent to the nanopore sequencing device to continue the sequencing of the biopolymer fragment in the nanopore of the nanopore sequencing device for a defined length of building blocks, preferably at least 100 building blocks, and

receiving data from the nanopore sequencing device representing the elongated sequence of the biopolymer fragment, and

comparing the received elongated sequence to sequences comprised by the decoy sequence database and/or to sequences comprised by the target sequence database according to step ii., wherein one of alternative steps ii. a., b. or c. is performed,

iii. optionally determining the presence of one or more target sequences in the sample according to step ii.b.

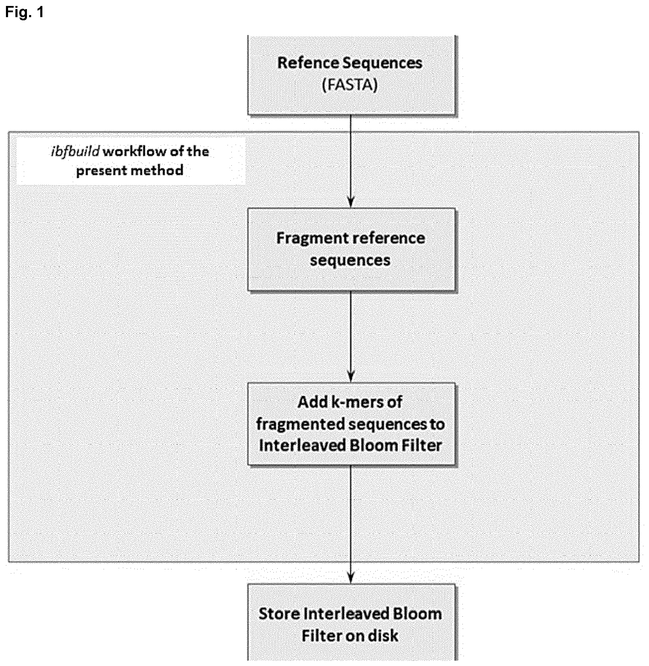

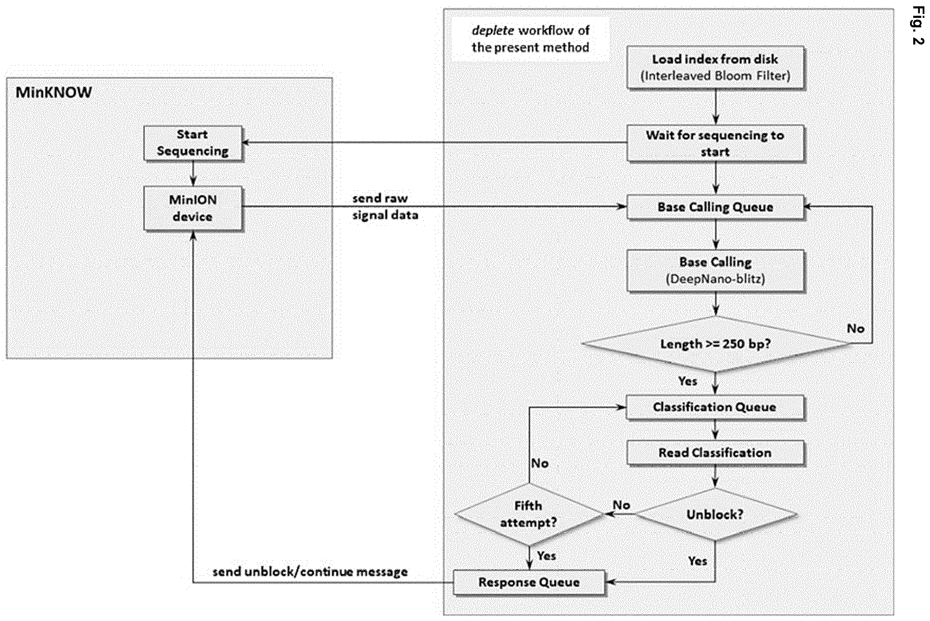

In one embodiment of the present invention, wherein the biopolymer analyzed by Nanopore sequencing is nucleic acid and which is also shown in Figure 1, the reference sequences are loaded from a FASTA file and splitted into fragments of equal size. Subsequences, such as k-mers, of the fragments are added to the probabilistic data structure, which is an interleaved Bloom filter (IBF) in this embodiment, which is finally stored as an index file on the hard disk. In one embodiment of the present invention, which is also shown in Figure 2, subsequently the probabilistic data structure, which is an interleaved Bloom filter in this embodiment, of reference sequences is loaded from index file first. The present method then comprises a pause until the MinKNOW software of the nanopore sequencing device starts the sequencing run. When sequencing has begun, the MinION nanopore sequencing device sends raw signals for every DNA molecule currently traversing a nanopore to the computer performing the method according to the invention, which pushes the signals onto a base-calling queue. The base-calling thread takes signals from the queue and performs base calling. Base called sequence is pushed onto the classification queue if the length is equal to or longer than 250, preferably at least 100 nucleotides or pushed back to base calling queue otherwise. The classification thread takes sequence from the classification queue and queries the sequence against the target and/or decoy IBF. If the sequence is only found in the decoy IBF, the read is marked for unblocking and pushed onto the response queue. If the sequence is neither found in the target IBF nor the decoy IBF, or found in both the target and the decoy IBF, the read is pushed back to the classification queue. One specific embodiment of the present method aims to classify a read five times using consecutive chunks of data. If the read is not unequivocally classified as match to the decoy IBF for the fifth time, the classification of that read is stopped and the read is marked for sequencing as usual. A *stop_further_data* message for that read is pushed onto the response queue. Finally, the response thread sends back action messages of reads from the response queue to the MinKNOW software and MinION device, respectively.

The invention also relates to a data processing apparatus comprising means for carrying out the computer implemented method according to the invention.

The invention also relates to a computer program [product] comprising instructions which, when the program is executed by a computer, cause the computer to carry out the computer implemented method according to the invention.

The invention also relates to a computer-readable medium having stored thereon the computer program [product] according to the invention.

The present method offers a new approach for nanopore adaptive sampling. For this purpose, in one embodiment the method according to the invention implements the entire analysis workflow in one software package without third-party tool installation or package manager requirements. Embodiments of the present method facilitate real-time nanopore adaptive sampling even on general-purpose CPU-based systems by combining CPU-based base calling with compact and/or efficient indexing methods. One specific embodiment might use fast CPU base calling in combination with a dynamic searchable parallel compressed index (DREAM), to make fast ejection decisions. The present probabilistic data structure allows for fast querying of hashed subsequences, such as k-mers, on large sequence datasets and is easily adjustable for different tasks. Embodiments of the present method uses the IBF to classify base called DNA fragments for ejection and communicate the decision to the sequencing control software.

In comparison with the prior art tool "Readfish", the method according to the invention can make faster rejection decisions than Readfish, wherein the average read length of ejected nanopore reads is significantly smaller with the present invention than for Readfish. The present Examples provide evidence of the improvements the present method can provide over the prior art tool Readfish. Further, the read classification approach according to the present invention offers, compared to other software tools in a nanopore adaptive sampling context, superior accuracy, recall, F1-Score, and Matthews correlation coefficient (MCC) among all tools on simulated and real-world datasets, while having almost the same precision and specificity as the best prior art competitor method Readfish.

Furthermore, the present method also has the smallest reference sequence index size and peak computer memory usage. Finally, when the potential enrichment of low abundance organisms that can be reached by applying nanopore adaptive sampling using the present method is compared with Readfish, the present method achieves in embodiments up to 50% better enrichment in an experimental setup where up to 99.99% of sequenced reads shall be rejected. The present method also accomplishes better results than existing tools for adaptive sampling, even on a low-cost laptop without accelerated hardware, making it particularly applicable for in-field researchers and small sequencing laboratories with a small budget.

There are several use cases for which nanopore adaptive sampling is beneficial. First, the depletion of known contaminants in a sample can increase the number of sequenced reads of interest. Second, targeting specific gene regions (and/or specific regions in polypetides) with adaptive sampling can increase the sequencing depth at those regions of interest to identify single-nucleotide or single-amino acid variants and structural variations within the analyzed biopolymer sequences. Hence, in-silico enrichment of regions of interest can supersede the design of costly laboratory enrichment panels.

However, one of the most important applications is probably the sequencing of metagenomics samples, where enrichment of low-abundance genomes or proteomes can reduce the time and costs required to answer a biological question (time-to-answer) and improve the ability to assemble these low copy genomes or proteomes. This is especially useful for pathogen detection in human or animal samples that suffer from up to 99.99% of sequenced reads being host background.

This capability can, for example, make a significant contribution to the field of pathogen detection in non-model organisms, as missing reference sequences of the host animal under investigation can be overcome by using a reference sequence of a closely related species. In the present example, this scenario is mimicked by querying *rattus norvegicus* genome against the reference genome *of mus musculus,* wherein it could be demonstrated that the present read classification strategy with probabilistic data structures, such as interleaved Bloom filters, interleaved XOR filters or interleaved binary fuse filters, works significantly better than competing tools for this kind of application. Here, the CPU-based approach according to embodiments of the invention also gives access to adaptive sampling much easier for researchers studying wild living animals in the field.

Another potential application for adaptive sampling according to the invention is the real-time detection of antibiotic resistance and virulence genes and/or the downstream protein variants they translate to.

The present method facilitates, for example, these applications of adaptive sampling. Adaptive sampling comprises the depletion of host background-reads or sequences considered contamination from human or animal samples to reduce the time to detect a pathogen (target) and enrich the number of sequenced reads of that specific pathogen. Therefore, in embodiments of the method according to the invention nanopore reads are queried (compared) against large reference genomes or proteomes (provided in decoy database). By design, adaptive sampling does not require to query complete nanopore reads against the reference but only the prefix part that is sequenced during the first few seconds after the biopolymer molecule entered the pore. In embodiments of the invention this prefix is then classified as belonging to a region of a biopolymer sequence of interest or a depletion target region of a biopolymer sequence. In some embodiments reads from regions of interest shall be sequenced as usual, while reads from depletion targets are rejected. In general, the faster and more accurately the reads are classified, the faster uninteresting reads can be rejected, which results in a shorter time-to-answer and higher enrichment of interesting regions. Thus, in some embodiments the classification step in conjunction with the base-calling step needs to be fast enough to decide whether to eject a biopolymer molecule from the pore or continue sequencing as usual.

Moreover, in specific embodiments the improved classification algorithm of the present method achieves a 50% higher enrichment factor compared to the best prior art competitor, e.g. Readfish. Having 50% more reads of a low abundance organism can significantly impact downstream bioinformatics pipelines that try to assemble respective genomes or proteomes, or find antibiotic resistance genes or the protein variants they translate to. In these scenarios, the present method represents a substantial improvement to state-of-the-art methods.

The present invention makes it possible to perform genomic or proteomic pathogen diagnostics at low cost and to determine, for example, pathogen strains, resistant hospital germs or general resistance potential of an infection at the point-of-care (POC), e.g., in a doctor's practice or a retirement home, in near real time. Besides the detection of the presence of a pathogen, potential resistance or sensitivity to certain substances can be detected at the same time and before prescribing an antibiotic or before admitting or transferring a patient. This facilitates appropriate, targeted antibiotic administration or isolation of patients and helps reducing the spread of resistances and dangerous pathogens. This can entail massive cost reductions in the healthcare system and spare affected patients great suffering. In addition, due to the non-specific nature of the sequencing process itself, adaptation of the diagnostic spectrum is possible entirely in the used bioinformatics or software implementing the method according to the invention- without changes to the device or the chemicals used. Thus, under changed conditions (e.g., a new pandemic with a previously unknown pathogen or detection of a specific genotype not comprised by the reference databases), the present method and the used databases can easily be adapted to diagnose (new) relevant pathogens by a computational amendment or update.

In some aspects of the present invention, the present approach may also be applied in embodiments to analyze amino acid sequences. Accordingly, in such embodiments the amino acid sequence may preferably also be determined, under step i. by Nanopore sequencing, or in less preferred embodiments by other adequate means, such as mass spectrometry or other protein-sequencing methods. The amino acid sequence may then be analyzed according to steps ii. and iii., by comparing and matching the determined amino acid sequence using the present probabilistic data structure, approach and amino acid subsequences (such as k-mers) followed by comparison to target and/or decoy sequence databases (accordingly comprising amino acid (reference) sequences) to determine the identity of the analyzed amino acid. Accordingly, any embodiment described herein for the analysis of biopolymers, or for nucleic acid sequences might be applied in other embodiments of the invention for the analysis of amino acid sequences and vice versa.

The embodiments described herein for one aspect of the invention may also be embodiments of any one of the other aspects of the present invention. Accordingly, embodiments described for the method according to the invention may also be embodiments of the device, the computer implemented method and the computer program product disclosed herein. In addition, any embodiment described herein, may also comprise features of any other embodiment of the invention.

## DETAILED DESCRIPTION OF THE INVENTION

All cited documents of the patent and non-patent literature are hereby incorporated by reference in their entirety.

The present invention is directed to a method for determining the presence of one or more target sequences in a biopolymer-comprising sample using nanopore sequencing by taking advantage of the adaptive sampling feature of nanopore sequencing and by applying a new probabilistic data structure approach for biopolymer sequence comparison.

In general, the term "biopolymer" comprises biological polymeric molecules such as nucleic acids and proteins. In preferred embodiments of the invention the term biopolymer comprises exclusively nucleic acids or peptides/proteins. In other words, in preferred embodiments the term biopolymer herein refers exclusively either to nucleic acids or to peptides/proteins. Biopolymers are usually built from or consist of molecular organic building blocks or short herein "building blocks". Biopolymers may in embodiments herein refer to nucleic acids or "polynucleotides", e.g. DNA or RNA, which consist of a consecutive sequence of building blocks, namely nucleotides (nucleotide monomers). Biopolymers may, in embodiments herein, refer to peptides, polypeptides or proteins, which usually consist of a consecutive chain of building blocks, namely amino acids. Accordingly, herein, depending on the embodiment and the biopolymer analyzed in said embodiment a building block of a biopolymer can be a nucleotide in case of nucleic acids, such as DNA or RNA. In other embodiments, where the biopolymer is a peptide, polypeptide or protein a building block is an amino acid. It is understood, that in the context of the present invention the term building block, e.g. in a chain or sequence of one or more consecutive building blocks, may refer to any type, namely the same type or different types, of nucleotides or amino acids.

Herein, the term "nucleic acid" (in embodiments herein also referred to as biopolymer) may preferably refer to DNA (deoxyribonucleic acid), gDNA (genomic deoxyribonucleic acid), cfDNA (cell free DNA), RNA (ribonucleic acid), gRNA (genomic ribonucleic acid), mRNA (messenger ribonucleic acid) and cDNA (complementary deoxyribonucleic acid synthesized from RNA template), or any combination thereof. Herein nucleic acid sequences may be genomic nucleic acid sequences, mitochondrial nucleic acid sequences, transient nucleic acid sequences, vector nucleic acid sequences, exogenous nucleic acid sequences, artificially introduced nucleic acid sequences, endogenous nucleic acid sequences or any combination thereof.

Nucleic acid sequences refer herein to a consecutive array of nucleotides (in embodiments herein also referred to as building blocks of (nucleic acid-) biopolymers), wherein different (types of) nucleotides are distinguished by their nucleobases into guanine (G), adenine (A), cytosine (c) and thymine (T) in DNA and uracil (U) -instead of thymine- in RNA. A nucleic acid sequence may herein also refer to the sequence of consecutive letters (in case of the four canonical nucleobases of DNA/RNA G, A, C and T or U) that represent the actual sequence of consecutive nucleic acids in a strand of DNA or RNA. This nucleic acid sequence may be biochemically and bioinformatically identified and characterized using DNA or RNA sequencing. Nucleotide modifications (chemically modified nucleotides of DNA or RNA) may also be identified herein using sequencing. In preferred embodiments herein, the sequencing of nucleic acid or DNA/RNA is nanopore sequencing. The sequencing analysis may also involve the comparison of the obtained nucleic acid sequence and one or more reference nucleic acid sequences.

Herein the term "peptides" refers to short chains or sequences of (consecutive) amino acids, which are linked by peptide bonds. Longer chains of peptides, so called "polypeptides" can also be referred to as proteins, wherein proteins can consist of one or more (poly)peptides arranged in a biologically functional way. Proteins or (poly)peptides can be classified as biological polymers (in embodiments herein also referred to as biopolymer), alongside e.g. nucleic acids.

Peptides, polypeptides and proteins consist of amino acids (in embodiments herein also referred to as building blocks of (polypeptide-) biopolymers). In general, amino acids are organic compounds that contain amino ($-NH^+_3$) and carboxylate ($-CO^-_2$) functional groups, along with a side chain (R group) specific to each type of amino acid. When amino acids are incorporated into peptides or proteins they can be referred to as "residues" or amino acid residues. During this incorporation reaction a water molecule is released, and an amide bond is formed, thereby connecting two amino acids in a peptide/protein chain or sequence. Peptides (except cyclic peptides) generally comprise an N-terminal amine group and a C-terminal carboxyl group residue at the respective end. When referring in embodiments herein to a "peptide sequence", also a polypeptide sequence or protein sequence, namely a sequence of consecutive amino acids, may be meant. Accordingly, in embodiments of the invention the terms polypeptide and peptide may be used interchangeably. Further, in embodiments of the invention the terms polypeptide and protein may be used interchangeably.

In the context of the present invention, the term "sequence" can in embodiments refer either to a nucleic acid sequence or to an amino acid sequence, depending on the biopolymer that is analyzed or referred to in that specific embodiment.

Herein, the exact sequence of consecutive nucleotides or amino acids of a biopolymer, e.g. a nucleic acid or peptide/protein, may also be referred to as the "sequence identity" of said biopolymer, e.g. nucleic acid. A shared sequence identity means herein that, for example in case of nucleic acids, two nucleic acids share the exact same sequence of a certain number of consecutive nucleotides, which may also be referred to as 100 % sequence identity. The same applies for peptides/proteins sharing the exact same sequence of a certain number of consecutive amino acids. Lower percentages of sequence identity relate to a non-perfect sequence identity of the nucleic acid or amino acid sequence, wherein the indicated percentage of the sequence is identical.

"Base calling" in general and herein refers to the conversion of nucleic acid sequencing raw data to nucleic acid sequences. In other words, in the context of the nanopore sequencing process of the method of the invention base calling is the process of assigning nucleobases to electrical current changes resulting from nucleotides passing through a nanopore. In one embodiment base callers for nanopore sequencing use neural networks trained on current signals obtained from real sequencing data. Base calling is preferably assessed by two metrics, read accuracy and consensus accuracy. Read accuracy refers to how accurate the called base is to a known reference. Consensus accuracy refers to how accurate a consensus sequence is compared to overlapping reads from the same genetic locus. A similar approach to base calling can in embodiments be applied for the identification of sequenced amino acids, based on a characteristic electrical current change detected in a nanopore induced upon the passage of respective amino acids.

"Nanopore sequencing" describes a sequencing method involving a sequencing device equipped with nanopores. The principle of nanopore sequencing is a device comprising a membrane to which a current is applied, while the membrane comprises a multitude of nanopores, wherein, when a potential is applied across a nanopore, the current flow changes when a nucleic acid or an amino acid passes through or resides within the pore for a certain period of time. The sequencing, namely the determination of the identity of consecutive nucleotides in a nucleic acid fragment or of consecutive amino acids in a (poly)peptide/protein, is achieved, as each nucleotide or amino acid causes a characteristic current change of known signature and duration. In nanopore sequencing a single polynucleotide or (poly)peptide strand passes through a nanopore, wherein the identity of each consecutive nucleotide of the polynucleotide, or each consecutive amino acid of the (poly)peptide, is determined, based on the characteristic change in the current flowing through the nanopore-comprising membrane. Nanopore sequencing might, in one embodiment of a nanopore device, involve a nucleotide handling protein (e.g., helicases), which resides at each nanopore and controls the stepwise movement of the polynucleotide through the nanopore, and optional also controls the recruitment and attachment of the nucleic acid to the nanopore before the sequencing begins. Similar or equivalent mechanisms may in embodiments also be applied for peptide sequencing.

A nanopore sequencer is a single-molecule, long-read sequencing device that can directly sequence RNA, DNA as well as polypeptides. A nanopore sequencer can also detect modifications of DNA and RNA molecules. Nanopore sequencing can, for example, also identify DNA and RNA base modifications at nucleotide resolution, including 4-methylcytosine (4mC), 5mC, hydroxymethylcytosine (5hmC), 5-formylcytosine (5fC), 5-carboxycytosine (5caC), N-6-methyladenine (6mA), and BrdU in DNA, and m6A in RNA. Hence, in some embodiments of the present invention also the analysis and identification of nucleotide modifications or sequences comprising nucleotide modifications is desired and can be implemented. As modified nucleobases comprise their own specific current signature in Nanopore sequencing, specific base calling algorithms capable of identifying and classifying nucleotide modifications might be used in such approaches.

Nanopore sequencing directly detects nucleotides without active DNA synthesis, while the single stranded DNA or RNA passes through a protein nanopore that is stabilized in an electrically resistant polymer membrane of the sequencing device. By setting a voltage across this membrane, sensors detect the ionic current changes shifted by nucleotides occupying the pore in real time as the DNA molecule passes through. Nanopore sequencing does not require imaging equipment to detect the nucleotides, allowing the system to scale down in size to a portable level.

A variant of the MinION nanopore sequencing device only weighs about 90 g, measuring only about 3.3 x 10.5 x 2.3 cm in size. In the embodiment of specific nanopore sequencers, such as the MinION device, the electric current is measured at each nanopore thousand times per second while single stranded DNA or RNA fragments move through the nanopore, which causes changes in the electric current. The electric current depends mostly on the context of several nucleic acid bases passing through the pore at the time of measurement. As the DNA or RNA moves through the pore, the context shifts and the electric current changes. Based on these changes, the sequence of measurements is split into events, each event ideally representing the shift of the context by one base. Each event is summarized by the mean and variance of the current and by event duration. This sequence of events is then translated into a DNA or RNA sequence by a base-calling algorithm. The cost of such a device is also much lower compared to other massively parallel sequencers, the initial cost being only around $1,000, including the device and initial set of reagents. MinION devices can be powered through the USB port of laptop computers, such that sequencing can be conducted anywhere, even in the field. Lack of an image analysis step also allows real time base calling during sequencing, realizing rapid detection of target DNA or RNA for the screening of pathogens from clinical samples, for example. Moreover, since nanopore sequencing directly detects the input molecule without DNA amplification or synthesis, there is no apparent limit to the length of DNA that can be sequenced.

The challenge in read length using nanopore sequencers therefore is not in the sequencing technology itself, but in the library preparation step, which needs to extract and load intact extremely high molecular weight DNA into the flow cell of the sequencer. Sequenced reads of extremely long stretches of the DNA molecules exceeding a mega-base can be achieved. The extreme long reads enable *de novo* genome assembly without preparation of complex mate-pair libraries typically required with short read sequencing. The extremely long reads are also useful to determine sequences of genomic region containing long repetitive sequences, e.g., at centromeres or transposons, which is difficult with short read sequencing and where short reads cannot be mapped uniquely. Long reads further facilitate the study of structural variations within the genome. In addition, detection of the ionic current changes shifted by the nucleotides passing through the nanopore is not limited to the canonical four nucleobases of adenine, guanine, cytosine and thymine.

Taking advantage of the nature of unamplified direct sequencing, nanopore sequencing can directly observe base modifications such as methylation, and even direct sequencing of RNA molecules containing uracil bases. The excellent portability of a MinION device that can be powered from a mobile PC can make a significant contribution in the context of in-the-field sequencing. Moreover, the real time nature of the device, where DNA molecules passing through the pore are immediately base-called on a per-read basis as they are sequenced, becomes a more rapid and highly robust alternative to conventional clinical testing such as chemiluminescence, ELISA, immune chromatographic tests (ICT), and real-time PCR. In nanopore sequencing the accuracy of the sequencing depends on both the pore chemistry and the base-calling algorithm. The sequencing accuracy can also be improved by optimizing pore proteins used for the homopolymers (Kono et al., 2018).

Nanopore sequencing also facilitates single-molecule protein or peptide sequencing. Brinkerhoff et al. 2021(1) and (2), recently developed an approach in which a protein can be sequentially sequenced in single-amino-acid steps through a nanopore. During said sequencing different amino acids along a protein/peptide sequence can be identified through characteristic ion current changes induced in a nanopore. In some exemplary embodiments this can be achieved by linking a (poly)peptide or protein sequence to a nucleic acid molecule, which is the pulled through a nanopore, e.g. by a DNA helicase, in single amino-acid steps. In some embodiments multiple-read-coverage of the sequence of a protein/peptide molecule can be achieved by rewinding (rereading) the peptide or protein sequence inside a nanopore, thereby significantly reducing the error rate of single amino acid identification. Other approaches, mechanisms and/or method steps of sequencing a polypeptide inside a nanopore and/or using a Nanopore sequencing device are also envisaged in the context of embodiments of the present invention.

Different embodiments of preparing a nucleic acid nanopore sequencing library are disclosed in the following, wherein a final nanopore sequencing library must at least comprise a plurality of DNA, cDNA or RNA fragments, wherein at least a certain percentage of fragments is ligated each to a sequencing adaptor, which is configured to enable nanopore sequencing. In a preferred embodiment the plurality of DNA, cDNA or RNA fragments are first ligated each to at least one tag or "first" adaptor, and subsequently sequencing adaptors are ligated to said tag or "first" adaptors. Said tag or first adaptor should hence facilitate the attachment of a sequencing adapter and may optionally comprise binding sites for PCR and / or RT-PCR primers and/or may comprise a barcode sequence and/or tags enabling purification or enrichment.

RNA sequencing can be achieved by nanopore sequencing, for example, in embodiments either by direct RNA sequencing, e.g., when RNA modifications are of interest, or by reverse transcribing the RNA into DNA before sequencing. During sequencing library preparation, depending on the amount of RNA available, an optional poly-A based enrichment step can be performed. In some embodiments cDNA can be reverse transcribed from the RNA template by the means known in the art using a reverse transcriptase enzyme. Briefly, to a full-length messenger RNA a complementary DNA (cDNA) strand is synthesized using an oligo adapter comprising a poly-A sequence. The RNA is then degraded, and the second cDNA strand synthesized. Adapters are then ligated onto the cDNA, which introduce the components needed for the cDNA strands to enter the pore. One strand of the duplex is sequenced at a time.

In one embodiment an RNA sequencing library may be prepared for direct RNA sequencing after isolation of RNA from a sample. Isolated full-length RNA is ligated to double-stranded DNA adapters with a poly(T) or sequence-specific complementary single-stranded overhang to the 3' ends of extracted RNA molecules. Afterwards, an optional reverse-transcription step can be performed to linearize and stabilize the template RNAs. This optional cDNA strand is not sequenced, it only serves to stabilize the RNA until sequencing in the pore is performed. Finally, sequencing adapters are ligated to the double-stranded DNA adapters. Subsequently, the library is loaded into a nanopore sequencer for sequencing. In direct RNA sequencing, only the native RNA passes through the nanopore sensor thus the read length reflects the length of the RNA molecules in the sample.

In one embodiment a DNA sequencing library may be prepared after isolation of DNA from a sample. In one embodiment high molecular weight DNA can be optionally fragmented before the ends of each DNA molecule are end-repaired and/or nick-repaired and/or dA-tailed. In one embodiment then PCR adapters, which contain primer binding sites are ligated onto the prepared ends and an amplification step is performed using primers that contain 5' tags which facilitate the ligase-free attachment of sequencing adapters. Subsequently sequencing adapters are attached or ligated to the DNA molecules or fragments, preferably to the PCR adapters.

In another embodiment of preparing a sequencing library high molecular weight DNA may be cleaved with tags being ligated at the same time using transposase complexes. Subsequently sequencing adapters may be ligated to the tags. In one embodiment DNA molecules are first enzymatically end-prepared and the resulting DNA is purified. In one embodiment the DNA is afterwards optionally barcoded by ligation of a barcoding adaptor. Finally sequencing adaptors are ligated to the optionally barcoded DNA.

Optionally the library preparation comprises one or more washing or purification steps. In each embodiment the final step is the loading of sequencing-adapter-ligated DNA or RNA molecules (library) onto the nanopore sequencer.

In one embodiment the samples may also be processed to a sequencing library by using a VolTRAX or comparable device for automated sample preparation and sequencing library construction.

Herein a "host" or a host species refers to a species from which a sample is taken, and/or whose nucleic acid and/or proteins makes up the majority of a biopolymer-comprising, e.g. nucleic acid-or protein-comprising, sample. Possible hosts of the present invention can be selected from the group of bacterial, viral, fungal, protist, plasmodia, eucaryotic, amphibian, avian, plants, preferably mammal or human. Herein the terms "subject", "patient" or "host" may be used interchangeably. Herein as subject, patient or host may be an organism, a cell culture of patient cells or cell lines, an animal, or a cell culture of animal cells or cell lines. In case of an environmental sample, "host" sequences may in one embodiment be sequences that are not of interest, for example, from organisms that are not of interest, while target sequences may in one embodiment represent sequences of interest, e.g. from organisms of interest. In one embodiment, for example in case of a water or soil sample, the "host" sequences may be microorganisms and/or plants or just biopolymer sequences, such as nucleic acid and/or amino acid sequences, that are not of interest and are considered to be, for example, a biological "background" or contamination, while specific plants and/or microorganisms, such as fungi, viruses or bacteria, or just biopolymer sequences, such as nucleic or amino acid sequences, may be considered of interest (target).

Herein a "sample" may be taken from a patient, a cell culture of patient cells or cell lines, an animal, or a cell culture of animal cells or cell lines of a biopsy, a blood sample, a tissue sample, an environmental sample, or a food-derived sample. Basically, any kind of sample that is suspected to contain biopolymer sequences, e.g. nucleic acid or peptide/protein sequences, of interest, such as target sequences, among other biopolymer sequences, e.g. nucleic acid or peptide/protein sequences, such as preferably host sequences, may be used.

Accordingly, a patient sample may comprise biopolymer sequences from the patient himself and of other organisms or microorganisms comprised within the patient or patient sample, e.g. pathogens. In terms of environmental or food-derived samples, the sample may comprise biopolymers, such as nucleic acids or peptides/proteins, from a plurality of different (microorganisms comprised within said sample, and not necessarily biopolymers, such as nucleic acids or peptides/proteins, from a "host". In this case the decoy biopolymer, such as nucleic acids or peptides/proteins, may be from species or (micro)organisms that are not of interest.

Herein "point-of-care" testing (POC or bedside testing) is defined as medical diagnostic testing at or near the point of care - that is, at the time and place of patient care. "Lab-on-a-chip" technologies are one of the main drivers of point-of-care testing, especially in the field of infectious disease diagnosis. These technologies enable different bioassays such as microbiological culture, PCR, ELISA to be used at the point of care. POC testing is often accomplished through the use of transportable, portable, and handheld instruments. The coupling of POC testing devices to mobile technologies, such as laptops, enables the health care provider to quickly acquire and receive patient test results from a POC test device and immediately make medical decisions at the site of patient care, without the time and cost intensive involvement of an external laboratory. A point-of-care device might, in one embodiment, also be used in the filed or on sites of livestock farming. Immediate analysis of biopolymer sequences, e.g. nucleic acid or amino acid sequences, in a sample, without the need to send samples to an external laboratory, would also be of advantage in these embodiments.

Herein the terms "target sequence", "sequence of interest" or "pathogen sequence" may be used interchangeably. Consequently, a "target database" or a "pathogen database" both refer to the database comprising biopolymer, e.g. nucleic acid or amino acid, sequence data representative of one or more organisms, microorganisms, pathogens or genotypes to be identified, enriched, sequenced and characterized as they are of interest.

A target database may also contain or be specific for mutated or variant sequences, such as mutated pathogen sequences, or specific pathogen variant (i.e. genotype of interest) sequences. For example, sequences that comprise or code for features that enable resistance to drugs or sequences that provide sensitivity to a certain substance. In embodiments such databases can be used for sequencing and identification of biopolymer sequences, such as nucleic acid sequences or protein/peptide sequences. Accordingly, such databases comprise in embodiments either nucleic acid sequences or amino acid sequences.

Herein the terms "decoy sequence", "host sequence" or "depletion sequence" may be used interchangeably. Consequently, a "host database", a "decoy database" or a "depletion database" all refer to the database comprising biopolymer, such as nucleic acid or peptide/protein, sequence data representative of one or more host organisms, organisms or genotypes to be depleted from the dataset, as they are not of interest and considered biological or host "background" and/or contamination.

Herein, the target database and the decoy database comprise biopolymer sequence information, such as nucleic acid or peptide/protein sequence information, that can serve as reference information to biopolymer sequences, e.g. nucleic acid or peptide/protein sequences, obtained by nanopore sequencing according to the present invention. This reference information can be used to identify, characterize and/or classify a biopolymer, nucleic acid or peptide/protein sequence obtained by sequencing herein. Therefore, both target and decoy databases may also be generally referred to as reference databases, and the sequences comprised therein may be generally referred to as reference sequences or reference biopolymer, nucleic acid or peptide/protein sequences.

Herein the term "dynamically indexable database" describes databases, such as a target database and a decoy database according to the method of the invention, whose content, i.e. the comprised biopolymer, e.g. nucleic acid or amino acid, sequence data, can be modified dynamically, i.e. while performing the method of the invention. The term dynamically indexable database implies that biopolymer sequences comprised therein can be added, modified or removed from the database during the sequencing and/or while analysis is performed. Accordingly, in the present method a reference database can be modified, for example, depending on the target organisms or target biopolymers, such as nucleic acids or peptides/proteins, detected during the sequencing run. This enables, in one embodiment, during the sequencing run the addition or removal of more specific sequence information on members of a certain target organism, species or pathogen family that is detected, or on specific mutations leading to substance sensitivity or resistance (e.g. susceptibility or resistance to a certain medicament, vaccination, antibiotic or virostatic) of a pathogen. For example, in one embodiment not every genotype of a pathogen family needs to be comprised by the initial reference database, as upon detection of a member of a certain pathogen family or species, based on a reference sequence that is representative for the whole family or species, more detailed information on every genotype comprised within said family or species can be added at any time during the sequencing run and/or analysis. A genotype of interest may be a pathogenic variant of a microorganism and/or a mutation encoding resistance or susceptibility to an anti-microbial, a vaccination, virostatic and/or antibiotic. In the context of the present invention in embodiments any genotype, mutation or gene-variant may be of interest. In one embodiment, also biopolymer sequences considered to be specific for a sample contamination can be added during the sequencing and/or analysis run to a reference database. Said reference database may be the decoy or the target database, depending on the biological question to be answered. Accordingly, due to this possibility of constant adaptation of reference data based on detected targets, the initial reference databases only need to comprise information representative of, for example, organism families, species or strains, resulting in a smaller database size initially and throughout the sequencing process. In the context of the present invention smaller reference databases result in fewer storage capacity being required on the used computing device. Accordingly, comparisons of the obtained sequences to reference sequences can also be performed in shorter processing time.

In the context of the comparing step of the method of the invention, it is determined whether an obtained sequence "matches" to a sequence of a sequence database. Herein, it is understood that "matching" means that a biopolymer, e.g. a nucleic acid or peptide/protein, fragment, currently sequenced by a nanopore sequencer, shares a specified minimum number of subsequences, such as k-mers, with at least one bigger fragment of the aforementioned sequence from the database. The term k-mer refers to a subsequence of k consecutive building blocks of a biopolymer sequence, such as a subsequence of k consecutive nucleotides of a nucleic acid sequence or a subsequence of k consecutive amino acids of a peptide/protein sequence. The method according to the invention subdivides every reference sequence in the database in smaller overlapping fragments that can have a size of 100,000 to 1,000,000 building blocks, such as nucleotides or amino acids, and counts the number of identical k-mers between the sequenced biopolymer, e.g. nucleic acid or peptide/protein, fragment and each of the reference fragments of the sequences comprising the database. If the number of identical subsequences, such as k-mers, between a reference sequence fragment and the sequenced biopolymer, e.g. nucleic acid or peptide/protein, fragment exceeds a certain threshold, they are considered as a match. Herein, the method according to the invention determines the threshold for the minimum required number of identical k-mers between the two biopolymer sequences, e.g. nucleic acid or peptide/protein sequences, based the maximum number of k-mers of the sequenced biopolymer, e.g. nucleic acid or peptide/protein, fragment and the expected sequencing error rate.

The method according to the invention offers the possibility of "adaptive sampling" during real-time nanopore sequencing. Herein, adaptive sampling describes the process, of determining with the method according to the invention, while the biopolymer strand is sequenced within an individual nanopore, whether a sequence represents a target or a decoy sequence. This is achieved through the fast matching of the first 100-4000, usually the first 100-500, building blocks of the biopolymer sequence to one or more reference database(s). If the acquired sequence matches to a target to be enriched, and/or does not match to a decoy sequence to be depleted, the sequencing of the biopolymer fragment is continued and preferably the entire fragment is sequenced. In some specific cases, where the sequence matches to a decoy and a target sequence, the sequencing may also be continued to acquire further sequence information of the respective fragment. If the sequence does not match to a target sequence, and/or is to be depleted (e.g. as it matches to a decoy sequence), the fragment is selectively discarded from that nanopore, preventing further sequencing and vacating the pore for a new biopolymer to be analyzed. This approach enables target enrichment and the depletion of off-target sequences (e.g., host sequences) during sequencing itself, with no requirement for upfront laboratory assay-based depletion or enrichment. This process of adaptive sampling in the context of the present method also facilitates the enrichment and therefore the detection of low-abundance sequences, e.g., originating from low-titer pathogen infections in a patient. The time-saving approach of adaptive sampling achieves in conjunction with the probabilistic data structure approach according to the invention the surprising effect of an even faster sequence analysis paired with improved sensitivity and a significantly reduced need for computational resources (use of a CPU instead of a GPU), which leads, for example, to improved and simplified pathogen detection in a sample.

Herein, a "bit array" or "bit vector" (also bit map, bit set, bit string,) can be described as an array data structure that compactly stores bits or as a mapping from some domain (almost always a range of integers) to values in the set {0, 1}. Hence, as there are only two possible values (0, 1), they can be stored in one bit. The most important thing here is that the values supplied by the hash functions are evenly distributed; cryptographic properties are not required. One non-limiting application of the use of bit arrays is the Bloom filter, a probabilistic (set) data structure that can store large sets in a small space in exchange for a small probability of error. It is also possible to build probabilistic hash tables based on bit arrays that accept either false positives or false negatives.

In the context of the present invention the terms "bin" or "binning bitvector" refer to a segment of the probabilistic data structure that is used as basic data structure for the target and decoy databases. In case of interleaved bloom filters the probabilistic data structure can be considered a set of one or more Bloom filters that are combined into one single Bloom filter. Herein, a Bloom filter is defined as a bit array with a corresponding set of hash functions. Initially, all bits within the bit array are set to 0 and when an element, e.g. a k-mer, is added to the Bloom filter an integer value is computed with each of the hash functions and the bit at the corresponding positions of the bit array is set to 1. The method according to the invention uses in embodiments applying an interleaved Bloom filter one bin for each fragment of the reference sequences and combines them into one single interleaved Bloom filter. This procedure offers the possibility to search for a single k-mer in all bins of the interleaved Bloom filter in parallel instead of querying every bin separately one after another.

A confidence interval (CI) is a range of values that is likely to include a value from a population (e.g., a pool of individuals from which a statistical sample is drawn) with a certain degree of confidence. A confidence interval may be expressed as a percentage (%), whereby the mean of a population lies between an upper and lower interval. The 95% confidence interval is a range of values that one can be 95% confident contains the true mean of the population. Due to natural sampling variability, the sample mean (center of the CI) may vary from sample to sample. In other words, a confidence interval (also called confidence interval, confidence range or expectation range) is an interval in statistics that is intended to indicate the precision of the position estimate of a parameter (e.g. a mean value). The confidence interval specifies the area that includes the parameter of a distribution of a random variable with a certain probability (the coverage probability). In case of a confidence interval of 95% -if the random experiment is repeated in an identical manner - a 95% confidence interval will cover the unknown "true" parameter in approximately 95% of all cases.

"Subsequences" may herein refer to "k-mers", which are substrings (sequences, series or chains of subsequent biological molecules that are a small part ("sub") or component of a larger molecule of consecutive biological molecules ("string") of length k contained within a biological sequence, preferably k-mers are composed of building blocks, such as nucleotides or amino acids (i.e., in case of nucleic acids A, T/U, G, and C or other nucleobases). The term k-mer refers to all subsequences of a sequence of length k, accordingly in the example of the nucleic acid sequence AGAT said subsequence would comprise four monomers (A, G, A, and T), three 2-mers (AG, GA, AT), two 3-mers (AGA and GAT) and one 4-mer (AGAT). In embodiments of the invention K-mers can also be composed of letters serving as amino acid abbreviations. In other words, in general a sequence of length $L$ will have $L-k+1$ k-mers and $n^k$ total possible k-mers, where $n$ is the number of possible monomers (e.g., four in the case of the canonical nucleobases of DNA). In other embodiments the term "subsequences" may in the context of the present invention refer to k-mers, and/or minimizers and/or strobemers.

Within the context of this invention, a minimizer is defined as the smallest k-mer in a pre-defined ordering (e.g. lexicographically or by hash value) of each k-mer in the window of fixed length w. For a biopolymer sequence, such as a nucleotide sequence, "minimizers" from sliding windows yield a compact representation that can be used in a wide range of genome or proteome analyses applications. On the other hand, a "strobemer" is defined as a set of linked k-mers, where each k-mer is extracted from a specified window in a given biopolymer, e.g. nucleotide or amino acid sequence. Thus, a strobemer can also be defined as a set of linked minimizers.

Herein a "hash function" describes a function that can be used to map data of arbitrary size to fixed-size values. The values returned by a hash function are called "hash values", hash codes, digests, or simply hashes. The values are usually used to index a fixed-size table called a hash table. In general, hash functions and their associated hash tables are used in data storage and retrieval applications to access data in a small and nearly constant time per retrieval. They require an amount of storage space only fractionally greater than the total space required for the data or records themselves. Hashing is a computationally and storage space-efficient form of data access that avoids the non-linear access time of ordered and unordered lists and structured trees, and the often-exponential storage requirements of direct access of state spaces of large or variable-length keys. Hash functions are an essential ingredient of the Bloom filter, a space-efficient probabilistic data structure that is used to test whether an element is a member of a set.

A "Bloom filter" is a probabilistic data structure that can be used to quickly determine, which data has already occurred in a data stream, and which is appearing for the first time. For this purpose, a suitable number of hash functions is used to create a fingerprint of the analyzed data set in a single-line hash table. A Bloom filter consists of an m-digit bit array (which is initially filled with zeros) and h different hash functions with a value range from 0 to m-1. Initially, the Bloom filter is assigned its vocabulary. To do this, h hash values are determined for the value to be stored using the h hash functions, where each of the h hash values determined represents a position in the bit array. Each of these positions is now set to 1, whereby it is irrelevant whether there was previously a 1 or a 0 at this position. If it is now to be checked whether an arbitrary value is contained in the vocabulary, its h hash values are again determined. If one of the h positions of the bit array of the filter contains a 0 where the hash of the value to be checked has a 1, it is certain that the value is not contained in the filter. If there is a 1 in each of the h places in the filter where the value to be checked also has a 1, it is very likely that the value was stored in the filter. However, a Bloom filter can never say with absolute certainty that a certain table of contents functional principle value is really stored, because due to collisions there is a certain risk to classify values erroneously as belonging to it (false positive classification). To insert a k-mer into a Bloom filter, the bit positions that correspond to the h hash values of the k-mer are set to 1, and a k-mer is considered present in the Bloom filter if all h positions return a 1 during the lookup phase. In the present case, all subsequences, such as k-mers, of a reference genome would be inserted into the Bloom filter and the subsequences, e.g. k-mers, of a nanopore read would be looked up for in that Bloom filter.

With the current nanopore sequencing speed of 450 nucleotides per second (in case of nucleic acid analysis), an adaptive sampling approach should make ejection decisions within 2 seconds after sequencing of a DNA or RNA molecule has started. This requires fast base calling and rapid and reliable classification of read fragments smaller than 500 nucleotides.

The inventors therefore reasoned that, if one wants to get higher enrichment, the classification sensitivity needs to be improved for the same read length. Sequence mapping according to prior art methods might be problematic if no good quality reference sequence is available for an organism whose genomic information should be comprised within the decoy database, like non-model or less intensively studied wildlife organisms. In such scenarios, the reference sequence of a closely related species might be used for read classification. Prior art read mapping methods implementing this approach would most likely fail to find reads that should be ejected from the pore. The present approach uses a probabilistic data structure approach.

A "probabilistic data structure" describes a group of data structures that use hash functions, which randomize and compactly represent a set of items. These data structures can be used for parallel query membership tests with errors that can be controlled by the size of the data structure and the number of used hash functions. In the context of the present invention, probabilistic data structures specifically refer to bitvector-based representations of hashed items. These data structures comprise interleaved Bloom filters, interleaved XOR filters (IXF) and interleaved binary fuse filters (IFF) as well as other equivalent filters.

An "Interleaved Bloom filter" (IBF or ibf) combines several Bloom filters (bins) in one single bitvector. The IBF can be divided into several consecutive subvectors, each having the size of the number of bins. Thus, the first subvector contains the first bit of each bin, the second subvector the second bit of each bin, and so on. According to the method of invention one bin in the IBF corresponds to one fragment of one reference sequence, and thus the size of each subvector corresponds to the number of fragments. In this way, it is straightforward to address the single bits of every bin when inserting the subsequences, e.g. k-mers, of the reference sequence fragments. If, for example, one of the hash functions returns a value of 67 for a given subsequence (e.g. k-mer) of the $5^{th}$ fragment of the reference sequence, the $5^{th}$ bit in subvector number 67 is set to 1. This results in a compact data structure by having only one single memory efficient bitvector.

When querying a read against the probabilistic data structure (i.e. comparing an obtained biopolymer sequence, e.g. nucleotide sequence, to a sequence database by applying an IBF, IXF or IFF approach according to the invention) in order to check if it maps to any of the fragments, every k-mer of that read is matched against the probabilistic data structure. That means in one embodiment that the following steps are performed for every k-mer of the aforementioned read: First, the h hash values are computed by utilizing the h hash functions and the corresponding h subvectors are retrieved. Then, a logical AND for the IBF (or XOR for IXF and IFF) is applied to the h subvectors resulting in a binning bitvector. In the IBF approach, we check whether a bit in the binning vector is set to 1, and consider the k-mer to be included in the reference sequence fragment corresponding to that bin. The $6^{th}$ bit in the binning vector set to 1 means that the $6^{th}$ bit in all h subvectors is also set to one, which indicates that the k-mer is a member of the $6^{th}$ fragment of the reference sequence. In the IXF and IFF approach, one only needs to check whether a particular subsequence of the binning bit-vector corresponding to the $6^{th}$ fragment of the reference sequence is equal to a fingerprinting function's result of that k-mer. Using this method allow for counting the number of matching subsequences (e.g., k-mers) to every bin of the probabilistic data structure and a read is considered a match with a reference sequence fragment if the threshold for minimum number of matching subsequences (e.g. k-mers) is exceeded for that fragment.

An interleaved XOR filter (IXF) combines several XOR filters (bins) in one single bitvector. The IXF can be divided into several consecutive subvectors, each having the size of the number of bins. In case of a 16bit IXF, the first subvector contains the first 16 bits of each bin, the second subvector the second 16bit of each bin, and so on. In the context of embodiments of the method of the invention one bin in the IXF corresponds to one fragment of one reference sequence, and thus the size of each subvector corresponds to the number of fragments times the number of bits used for the fingerprinting function. In this way, it is straightforward to address the bits of every bin when inserting the subsequences, e.g. k-mers, of the reference sequence fragments. In contrast to the IBF approach, setting the bits is more difficult. Here one starts with the fingerprinting function's result and the hash values computed for the subsequence. Using the hash values, 16bit subregions in the subvectors are identified and the bits of the subregions are set in such a way that a logical XOR of these 16bit subregions is equal to the result of the fingerprint function. This results in a compact data structure by having only one single memory efficient bitvector that is even smaller than that of the IBF.

An interleaved binary fuse filter (IFF) is a variation of the interleaved XOR filter, where an additional hash function applied to the subsequences helps to combine only segments of subvectors within a smaller range addresses in memory. In embodiments the construction of these filters can be twice as fast as the construction of XOR filters and needs 15% less space.

In one embodiment, when choosing the correct parameter value for k in a k-mer-based approach, larger values for k will result in more specific read classification results but will also fail to find many reads from the reference genome or reference proteome when the number of sequencing errors is high. In embodiments when trying to classify nanopore reads with error rates of about 10%, the value for k will hardly become bigger than 13.

Herein a "bitwise AND" is a binary operation that takes two equal-length binary representations and performs the logical AND operation on each pair of the corresponding bits, which is equivalent to multiplying them. Thus, if both bits in the compared position are 1, the bit in the resulting binary representation is 1 (1 x 1 = 1); otherwise, the result is 0 (1 x 0 = 0 and 0 x 0 = 0).

A "logical AND" herein relates to the meaning in logic and mathematics, where AND is the truth-functional operator of logical conjunction; the AND of a set of operands is true if and only if all of its operands are true.

Herein a "bitwise XOR" is a binary operation that takes two equal-length binary representations and performs the logical XOR operation on each pair of the corresponding bits. If both bits in the compared position are equal, the bit in the resulting binary representation is 0 (1 ^ 1 = 0 and 0 ^ 0 = 1); otherwise, the result is 1 (1 ^ 0 = 1 and 0 ^ 1 = 1).

A "logical XOR" herein relates to the meaning in logic and mathematics, where XOR stands for "exclusive or". The negation of XOR is the logical biconditional, which yields true if and only if the two inputs are the same.

Herein an "index" or "index files" describes the representation of a reference sequence database, which is stored as a file on a hard disk drive. In general, a reference sequence is transformed into a data structure that is space and memory efficient. It also serves to guarantee fast lookups of subsequences in the reference sequence index in order to answer a biological question more quickly. According to the method of invention, the index file stores the probabilistic data structure on the hard disk of a computing device.

In the present context, a "subvector" describes a well-defined segment of a probabilistic data structure. Since a probabilistic data structure is a bit array, a subvector of a probabilistic data structure is a bit array as well. Herein, the size of each subvector is defined by the number of bins n comprising the probabilistic data structure, such that each subvector consists of exactly n consecutive bits of the probabilistic data structure. Furthermore, the subvectors are nonoverlapping, which means, that every bit in the probabilistic data structure belongs to only one subvector.

In the context of the present invention an "probabilistic data structure approach" or "interleaved probabilistic filter approach" comprises all steps involved in utilizing interleaved Bloom filters (IBFs), interleaved XOR filters (IXFs), interleaved binary fuse filters (IFFs) as well as other equivalent filters for biopolymer sequence, e.g. nucleic acid sequence or amino acid sequence, comparison or querying of sequenced reads against a database comprising of one or more reference sequences. This includes the building of an interleaved probabilistic filter for the purpose of the invention by subdividing the reference sequences into overlapping reference sequence fragments and adding all subsequences, e.g. k-mers, of the fragments to the corresponding bin of the filter. It also includes all methods for determining if a sequenced read matches to one or more bins of the filter by applying confidence intervals for the number of erroneous subsequences, e.g. k-mers, in order to determine the threshold for the minimum number of matching subsequences, e.g. k-mers, required to consider the read a match.

Herein the "read until" approach describes a selective sequencing method, which can be performed on a nanopore sequencing device. As a biopolymer, such as a nucleic acid or polypeptide, transits a nanopore, it can be matched in real-time against sequences of a target and/or a reference database. Biopolymers of interest are allowed to continue to be sequenced while biopolymers with sequences that are not of interest are physically rejected from the pore, e.g. by reversing its polarity in case of nucleic acid-sequencing. Consequently, herein a "read until client" describes a specific piece of software that communicates with the sequencing device by receiving measured current signals for each biopolymer molecule from the device and sending response messages for each molecule traversing the nanopore back to the sequencing device. These response messages indicate whether a biopolymer molecule shall be ejected from the nanopore or sequenced as usual until the end of the molecule or sequence.

Herein an "ibfbuild workflow" describes the method of constructing an interleaved Bloom filter according to the invention. In one embodiment an ibfbuild workflow comprises the addition of reference sequences, such as decoy and/or target sequences, from FASTA-formatted sequence files, wherein the sequences are splitted into fragments of equal and specific size. The subsequences, e.g. k-mers, of the sequence fragments are added to the interleaved Bloom filter, which is in one embodiment finally stored as an index file on a hard disk of a computer or in another embodiment on a computer-readable storage device.

Herein the term "sequence alignment" describes the procedure of arranging biopolymer sequences, e.g. nucleic acid sequences, such as sequences of DNA or RNA, to identify regions of similarity that may be a consequence of functional, structural, or evolutionary relationships between the sequences. Aligned sequences of building blocks, such as nucleotide residues, e.g. represented by the letters A, G, T, C, U or N (for NULL, NA or not identified) representing the nucleobases of the nucleic acids, are typically represented as rows within a matrix. Gaps are inserted between the residues so that identical or similar characters are aligned in successive columns. Alignments are commonly represented both graphically and in text format. One approach of sequence alignment is pairwise sequence alignment, which aims to find the best-matching piecewise (local or global) alignments of two query sequences. Multiple sequence alignment is an extension of pairwise alignment to incorporate more than two sequences at a time. Multiple alignment methods try to align all of the sequences in a given query set.

Unless defined otherwise, all technical and scientific terms used herein have the same meaning as commonly understood by one of ordinary skill in the art to which this invention belongs. The practice of the present invention will employ, unless otherwise indicated, conventional techniques of chemistry, molecular biology, microbiology, recombinant DNA technology, and bioinformatics. Bioinformatics and sequencing terms, unless otherwise indicated herein, have the meaning as described, for example in Baxevanis et al.

**FIGURES**

The invention is further described by the following figures. These are not intended to limit the scope of the invention but represent preferred embodiments of aspects of the invention provided for greater illustration of the invention described herein.

Brief description of the figures:

**Figure 1:** Flow diagram of an ibf-build workflow of the method according to an embodiment of the invention.

**Figure 2:** Flow diagram of a deplete workflow of an embodiment of the method according to the invention.

**Figure 3:** Example of construction of a probabilistic data structure, here an Interleaved Bloom filter (IBF), according to an embodiment of the present invention.

**Figure 4:** Workflow of determining the correct fragment for a given read p. For each k-mer of read p, the three hash values are calculated using the same hash functions as for the IBF construction, as an example of an embodiment of a probabilistic data structure.

**Figure 5:** Read length distributions of unblocked reads when using a) an embodiment of the present method or b) Readfish on a 30 minutes playback of a whole human DNA sequencing run.

**Figure** 6: Proportion of reads from each species in the simulated mock community dataset. Less than 2.5% of reads were simulated from Saccharomyces cerevisiae. The aim would be to deplete bacterial reads in order to enrich the Saccharomyces cerevisiae.

**Figure 7:** Visualization of (a) Recall and (b) Specificity with varying simulated read accuracies for the present method, minimap2, and SPUMONI.

**Figure** 8: Proportion of reads from each species in the real mock community dataset.

Detailed description of the figures:

**Figure 1:** Flow diagram of the ibfbuild workflow of the present method. Reference sequences are loaded from a FASTA file and splitted into fragments of equal size. Subsequences, e.g. k-mers, of the fragments are added to the probabilistic data structure, here an interleaved Bloom filter, which is finally stored as an index file on the hard disk.
**Figure 2:** Flow diagram of the present method's deplete workflow. Probabilistic data structure, here an interleaved Bloom filter, of reference sequences is loaded from index file first. The present method then comprises a pause until the MinKNOW starts the sequencing run. When sequencing has begun, the MinION device sends raw signals for every DNA molecule currently traversing a nanopore to the computer performing the method according to the invention, which pushes the signals onto a base-calling queue. Base-calling thread takes signals from the queue and performs base calling via DeepNano-blitz. Base called sequence is pushed onto the classification queue if the length is equal to or longer than 250 nucleotides or pushed back to base calling queue otherwise. The classification thread takes sequence from the classification queue and queries the sequence against the IBF. If the sequence is found in the IBF, the read is marked for unblocking and pushed onto the response queue. If the sequence is not found in the IBF and corresponds to the first two chunks of the corresponding read, the read is pushed back to the classification queue. One embodiment of the present method aims to classify a read five times using consecutive chunks of data. If the read is not found in the IBF for the fifth time, the classification of that read is stopped and the read is marked for sequencing as usual. A stop_further_data message for that read is pushed onto the response queue. Finally, the response thread sends back action messages of reads from the response queue to the MinKNOW software and MinION device, respectively.
**Figure 3:** Example of the construction of a probabilistic data structure, here an interleaved Bloom filter (IBF) in an embodiment of nucleic acid analysis. In the first step, the reference sequence is subdivided into three overlapping fragments. Then, for each k-mer of the differently colored fragments, all three hash values have to be calculated. The resulting hash values determine the subvector SV3 in which the corresponding bit is set to 1. For example, the second hash function for k-mer AATTGTC from fragment F3 returns e. Hence, the third bit of subvector SVe was set to 1. In this way, the three Bloom filters for the three fragments are combined in an interleaved fashion. Since there are three fragments in this example, the length of every subvector is three, and the length of the IBF is 3x, where x is the defined length for every Bloom filter of the three fragments.

**Figure 4:** Example of the approach of finding the correct fragment for a given read p in embodiments of nucleic acid analysis according to the present invention. For each k-mer of read p, the three hash values are calculated using the same hash functions as for the IBF construction. The resulting hash values are used to find the corresponding subvectors of the IBF. The sub bitvectors are combined with a bitwise AND to a binning bitvector. For all set bits in the binning vectors of the subsequences, e.g. k-mers, the counter of the corresponding bin are incremented in a counting vector. Bins whose counter is greater than or equal to a given threshold t are considered to contain the read p. In this example, the calculation of the binning bitvector for the 7-mer CAGGATT is shown. Using the same three hash functions as for the IBF construction in Figure 4, the subvectors $SV_2$, $SV_k$ and $SV_x$ are obtained. These three subvectors are combined via logical AND to get the binning bitvector. The same procedure is applied to the other three 7-mers, and with the resulting four binning bitvectors, the number of matching 7-mers of read p can be calculated with each fragment. If at least three 7-mers match against one fragment, the read is accepted as a match with the reference sequence.

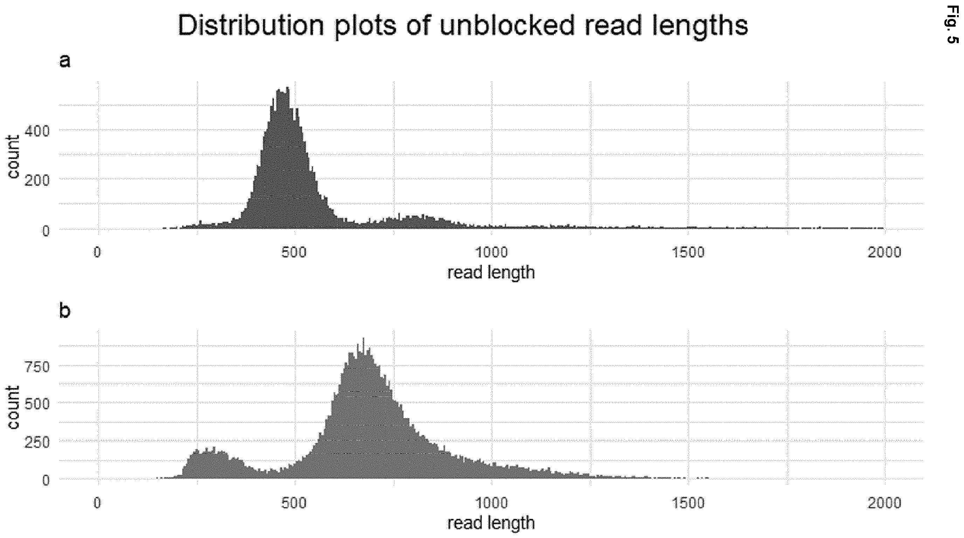

**Figure 5:** Read length distributions of unblocked reads when using a) embodiments of the present method or b) Readfish on a 30 minutes playback of a whole human sequencing run.

**Figure 6:** Example of the proportion of reads from each species in the simulated mock community dataset in an embodiment of the present invention. Less than 2.5% of reads were simulated from Saccharomyces cerevisiae. The aim might be to deplete bacterial reads in order to enrich the Saccharomyces cerevisiae.

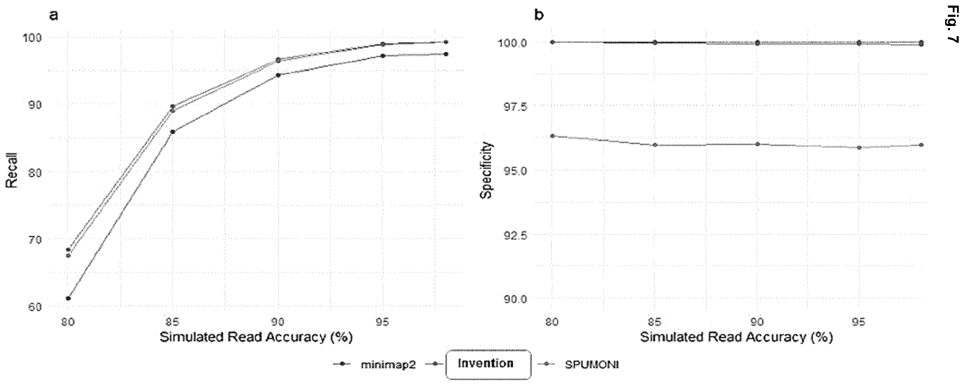

**Figure 7:** Visualization of (a) Recall and (b) Specificity with varying simulated read accuracies for embodiments of the present method, minimap2, and SPUMONI. All three tools show a high specificity among the different read accuracies, with the present approach and minimap2 showing almost perfect specificity. The sensitivity increases with the read accuracy for all three tools. The present approach is the only tool that combines high sensitivity with high specificity.

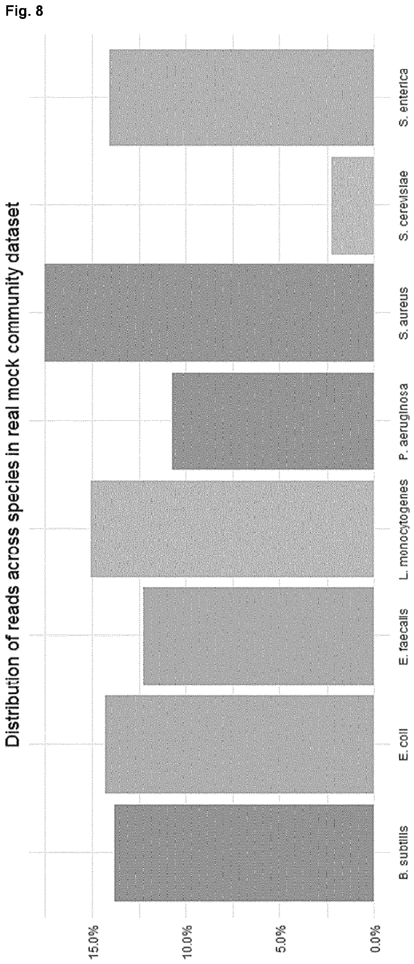

**Figure 8:** Example of the proportion of reads from each species in the real mock community dataset in an embodiment of the present invention. Only 2.5% of reads sequenced from a real mock community originate from Saccharomyces cerevisiae. The aim might be to deplete bacterial reads in order to enrich the Saccharomyces cerevisiae.

## EXAMPLES

The invention is further described by the following examples. These are not intended to limit the scope of the invention but represent preferred embodiments of aspects of the invention provided for greater illustration of the invention described herein.

## Methods according to the invention that were implemented in the Examples

Workflow

The workflow of the method according to the invention consists in one embodiment of nucleic analysis of two consecutive parts. First, an index of the given reference sequence data set is constructed and stored as a file on the computer hard disk (Figure 1). The construction of this index, for which probabilistic data structures, in the resent example an interleaved Bloom filter, are applied, is explained in further detail in section Read Classification. The second part of one particular embodiment of the method according to the present invention is the live-depletion/enrichment task (Figure 2), which starts with loading one or more index files from a hard disk (computer storage device). The next method step is the start of the sequencing process on the MiniON nanopore sequencing device. Immediately after sequencing has begun, the sequencer streams raw electrical currents for every single molecule from every single sequencing pore of the flow cell to our integrated "Read-Until" client. Oxford Nanopore provides this functionality via an Application Programming Interface (API) of its MinKNOW control software (Oxford Nanopore Technologies 2021), which allows our Read-Until client to receive the raw data while the molecule traverses through the pore. The client is implemented in C++ and communicates with the MinKNOW control software via gRPC remote procedure calls (https://github.com/grpc/grpc). Received raw signal data get pushed onto a base-calling queue, and a separate thread takes each reads raw signals from the queue and translates the electrical currents into a nucleotide string. In the herein applied embodiment the inventors integrated DeepNano-blitz (Boza et al. 2020) for this base-calling step, which is fast enough to perform the base calling in real-time, even on CPUs. In order to avoid reading and writing of intermediate files, the inventors integrated DeepNano-blitz by using the interoperability feature between the Rust programming language and C++ that makes it possible for us to call the functions from DeepNano-blitz directly. The inventors also integrated a software client in this embodiment that communicates with the state-of-the-art base calling software provided by Oxford Nanopore Technologies. This allows the utilization of more accurate base calling software in the presence of GPUs.

Base called reads get pushed to the classification queue if the read length is bigger than or equal to 250 nucleotides, and another thread takes each read from that queue and passes it to the classification framework. Otherwise, the thread marks this read as "pending" and waits for the following data chunk to be base called and concatenates the base called sequences of the read until the minimum read length has been reached. The minimum read length of 250 nucleotides ensures higher confidence in the classification of the reads. In practice, this read length requirement will lead to most reads having about 360 nucleotides length, which corresponds to two data chunks sent by the Min-KNOW nanopore sequencing software. The read classification thread then queries the read sequence against the loaded probabilistic data structure, here an interleaved Bloom filter, indexes as described in more detail in section Read Classification. Based on the classification, reads can either be marked for a rejection or continue further sequencing. If a read was not classified for rejection on a first try, it is marked as once_seen and the algorithm waits for further sequencing data to try further classification attempts of that read. After the read could not be classified for rejection for the fifth time, the read is marked for continued sequencing as usual. Reads that have been classified for rejection or continued sequencing are finally pushed to the response queue.

The last thread takes the classified reads from the response queue, and our Read-Until client sends response messages back to the MinKNOW control software for each read. The client sends an unblock message for reads that could be matched to the probabilistic data structure, here an interleaved Bloom filter, telling the sequencer to eject the corresponding DNA molecule. A stop_further_data message is sent to the control software for reads that were not classified for rejection. This message tells MinKNOW to continue sequencing the corresponding DNA molecule and send no additional chunks of data for that read.

Read Classification

With the current nanopore sequencing speed of 450 nucleotides per second, an adaptive sampling approach should make ejection decisions within 2 seconds after sequencing of a DNA molecule has started. This requires fast base calling and rapid and reliable classification of read fragments smaller than 500 nucleotides. The prior art method "Readfish" (Payne, Holmes, Clarke, et al. 2021) uses the long-read alignment tool "minimap2" (Li 2018) for this purpose. Although being fast and accurate for long error-prone nanopore reads, the alignment approach poses some challenges when working with short error-prone fragments of less than 500 nucleotides. For optimal enrichment of low abundance genomic regions, reliable rejection decisions need to be made as fast as possible. Payne et al. showed in their study that it needs about 360 nucleotides for minimap2 to align 90% of those reads correctly. The inventors therefore reasoned that, if one wants to get higher enrichment, the classification sensitivity needs to be improved for the same read length. Mappings are also hard to use when there is no good quality reference sequence available for an organism that is the depletion target, like non-model or less intensively studied wildlife organisms. In such scenarios, the reference sequence of a closely related species might be used for read classification. Mapping reads to the reference of a closely related species would fail to find numerous reads that should be ejected from the pore.

All these findings motivated the inventors to seek a different, fast classification strategy. The fastest current sequence comparison algorithms use k-mer based approaches, where a DNA sequence is divided into small overlapping substrings of size k. One prior art approach, known as "MinHash" (Broder 1997; Ondov et al. 2016), computes a hash value for every k-mer of a sequence and stores the smallest hash values within a data structure called a sketch. The same procedure is applied to the second sequence, and the number of hash values present in both sketches gives an accurate approximation of the identity between the two sequences. Although this works well for sequences of similar size, it fails for sequence containment tests, where one sequence is much smaller than the other one, which is the case when one wants to check if a nanopore read is part of a reference genome.

A better approach for testing if the set of subsequences, e.g. k-mers, of a reference genome contains the subsequences (e.g., k-mers) of a read is using Bloom filters (Bloom 1970; Koslicki and Zabeti 2019). A Bloom filter simply is a bitvector of size n and a set of h independent hash functions. To insert a k-mer into a Bloom filter, the bit positions that correspond to the h hash values of the k-mer are set to 1, and a k-mer is considered present in the Bloom filter if all h positions return a 1 during the lookup phase. In the present case, all subsequences (e.g., k-mers) of a reference genome would be inserts into the Bloom filter and the subsequences (e.g., k-mers) of a nanopore read would be looked up for in that Bloom filter.

The biggest problem of k-mer based approaches is choosing the correct parameter value for k, which is always a tradeoff between sensitivity and specificity in the presence of sequencing errors. Larger values for *k* will result in more specific read classification results but will also fail to find many reads from the reference genome when the number of sequencing errors is high. When trying to classify nanopore reads with error rates of about 10%, the value for k will hardly become bigger than 13.

The nanopore sequencing error rate was accounted for by the inventor in this example by choosing small k-mer sizes such as 13, which entails the need for smaller fragment sizes down to 100,000 nucleotides in order to avoid too many false positive matches. In the future, it can be expected that fewer fragments per genome might be used, which will consequentially improve the classification speed for larger genomes as Oxford Nanopore is steadily improving its per-read accuracy. For instance, with 95% raw read accuracy, a value of 19 for the k-mer size should be sufficient, and thus one might increase the fragment size in the present method to about 300,000,000 nucleotides. The present method might then only require around 11 bins in the probabilistic data structure, here an interleaved Bloom filter, which results in a much higher read classification throughput, even for human-sized genomes. This would also enable the usage of the the probabilistic data structure (here interleaved Bloom filter) approach according to the invention for real-time metagenomics classification of nanopore reads or the construction of pan-genomics indexes that store all different haplotypes of a pathogen in one probabilistic data structure, e.g. an interleaved Bloom filter, with one haplotype per bin. Thus, it is beneficial for this approach to use GPU-based base calling, which might also decrease the average read length of rejected reads and consequently reduces the time spent sequencing uninteresting or undesired nucleic acid molecules.

In the present example though, the number of different subsequences, here k-mers, of size 13 is combinatorial defined by $4^{13} = 67,108,864$, which is much too small when working with human-sized genomes that compose about 3 billion k-mers of size 13. To overcome this issue, the inventors decided to divide the reference genome into overlapping fragments of size m and construct a separate Bloom filter for each fragment. However, querying one read against each of the Bloom filters separately reduces the performance of the Bloom filter approach. Thus, the inventors decided to use probabilistic data structures as proposed by Dadi et al. 2018 for interleaved bloom filters to index the reference genomes.

An interleaved probabilistic filter or, e.g. interleaved Bloom filter, combines several probabilistic filters (bins) in one single bitvector. The interleaved probabilistic filter can be divided into several subvectors, each having the size of the number of bins. Since one bin in the interleaved probabilistic filter corresponds to one fragment of the reference sequence, the size of each subvector corresponds to the number of fragments.

In Figure 3, for example, the reference sequence was divided into three overlapping fragments, each corresponding to one bin of the probabilistic data structure, here an IBF. Thus, each subvector in the probabilistic data structure (here IBF) consists of 3 bits. The i-th bit of every subvector belongs to the Bloom filter bin of fragment $F_i$. When inserting a k-mer from fragment $F_i$ into the probabilistic data structure (here IBF), all h hash values were computed, which points to the corresponding subvectors $SV_j$ and then the i-th bit of this subvector was simply set to 1.

When querying a read p against the probabilistic data structure (here IBF) in order to check if it maps to any of the fragments, every k-mer of that read is matched against the probabilistic data structure (here IBF). That means the h subvectors $SV_j$ were first retrieved and a logical AND was applied to them, resulting in the required binning bitvector indicating the membership of the k-mers in the bins.

The example in Figure 4 visualizes this process. Here, the read consists of four 7-mers, for which the three hash values that point to the corresponding subvectors $SV_j$ have to be calculated, as can be seen in particular for the 7-mer CAGGATT. A logical AND of these three subvectors gives the binning bitvector for that 7-mer. In the present example, the binning vector 010 for CAGGATT tells that this 7-mer only matches fragment $F_2$. Applying this procedure to every 7-mer of the read provides four binning bit vectors. Finally, one only needs to sum up the 1-bits in the binning vectors for every fragment, which provides the number of matching 7-mers of the read for every fragment. Thus, instead of computing h hash values for every Bloom filter separately, one only needs to compute the h hash values once, which poses a significant reduction in computing time to investigate the membership of a k-mer in every Bloom filter. This method enables the user to quickly count the number of matching k-mers between the reference genome and a specific nanopore read. The challenge is to define a threshold value for the number of matching k-mers required to accept a certain nanopore read as a match against a fragment and thus as a match with the reference genome. In the present example in Figure 6, the inventors considered the read matching fragment $F_2$ because three of the four 7-mers match with that fragment. In general, the best threshold value depends on the length of the nanopore read and the expected sequencing error rate. In the next section, it will be described how the present method determines this value.

Optimal Bitvector Size

In a first step, the present method produces overlapping fragments of the given reference sequences, e.g., 100,000 nucleotide long fragments with an overlap of 1000 base pairs. Each of those fragments represents a single bin in the probabilistic data structure (here an interleaved Bloom filter). The constituting k-mers of each fragment are hashed using three different hash functions, and the bits of the corresponding index positions in the probabilistic data structure, here an interleaved Bloom filter, are set to one (Figure 3). Then, the present method automatically calculates the optimal probabilistic data structure (IBF) size in bits ($Bits_{IBF}$) based on the following equations.

$$Bits_{IBF} = n_{frag} \times Bits_{SBF} \qquad (5)$$

Where frag is defined as the number of fragments with maximum size F and Bits$_{SBF}$ as a single Bloom filter size for a single fragment. Let max$_{kmer}$ be the maximum number of k-mers for a fragment of size F, and k-mer size k be defined as

$$max_{kmer} = F - k + 1 \qquad (6)$$

To calculate the optimal size for the IBF, the inventors used the formula for finding the false positive rate in an probabilistic data structure (e.g. IBF) as proposed by Dadi et al. 2018.

$$p = \left(1 - \left(1 - \frac{1}{Bits_{SBF}}\right)^{h \times max_{kmer}}\right)^{h} \qquad (7)$$

Then the optimal size of a single Bloom filter can be calculated by resolving the formula for BitssBF:

$$Bits_{SBF} = \left\lceil \frac{-1}{(1 - r)^{\frac{1}{h \times max_{kmer}}} - 1} \right\rceil \qquad (8)$$

Where $r = p^{\frac{1}{h}}$, h is the number of used hash functions and p a predefined false positive rate. The present method implicitly uses three hash functions and a maximum false positive rate of 0.01 to minimize the number of false matches between the query sequence and a single bin of the probabilistic data structure, here an interleaved Bloom filter.

Minimum number of k-mer matches

During the read classification step, the k-mers of every read are hashed with the same three hash functions, and the number of matching k-mers for every bin is calculated as visualized in Figure 8. A read is accepted as part of the reference sequence if the number of matching k-mers is greater than or equal to a given threshold t for at least one bin. The threshold is calculated using the expected sequencing error rate e and the definition of a (1 - a) confidence interval of the number of erroneous k-mers as recently provided by Blanca et al. 2021. They first defined the expected number of erroneous k-mers as follows:

$$E[N_{err}] = L \times q \qquad (9)$$

For a given read $r$ with length $len(r)$ and k-mer length $k,$ the number of k-mers of read $r$ is denoted as $L = len(r) - k + 1$, and $q$ is defined by $(1 - (1 - e)^k)$. In a second step, they show that the variance for the number of erroneous k-mers can be calculated by

$$Var(N_{err}) = L(1 - q)\left(q\left(2k + \frac{2}{e} - 1\right) - 2k\right) + k(k - 1)(1 - q)^2 + \frac{2(1 - q)}{e^2}((1 + (k - 1)(1 - q))e - q) \qquad (10)$$

Finally, they define the (1 - a) confidence interval by:

$$E[N_{err}] \pm z_\alpha \sqrt{Var(N_{err})}$$

(11)

With $z_\alpha = \varphi^{-1}(1 - a/2)$,, where $\varphi^{-1}$ is denoted as the inverse of the cumulative distribution function of the standard Gaussian distribution. Based on the calculation of the confidence interval for the number of erroneous k-mers, the inventors defined the threshold for the minimum number of matching k-mers for read *r* as:

$$min[N_{match}] = L - \left(E[N_{err}] + z_\alpha \sqrt{Var(N_{err})}\right)$$

(12)

A read is classified as a match if the number of matching k-mers is bigger or equal to $min[N_{match}]$ for at least one bin in the IBF. In one embodiment the present method per default calculates this threshold for a 95%-confidence-interval, an expected sequencing error rate of 10%, and k-mer length 13. However, these values are adjustable via configuration parameters of the command line or graphical user interface (GUI).

DNA Sequencing

ZymoBIOMICS HMW DNA Standard was purchased from Zymo Research, from which 400 ng were transferred into a 1.5 mL DNA LoBind tube, and the volume was adjusted to 7.5 μL with Nuclease-free water. The sample was mixed by flicking the tube, spun down briefly, and then transferred into a 0.2 mL PCR tube. 2.5 μL Fragmentation Mix was added, and the tube was mixed by flicking the tube and spun down. After incubating for 1 min at 30°C and subsequently for 1 min at 80°C, the tube was briefly put on ice. 1 μL RAP was added to the barcoded DNA. After mixing by flicking the tube and spinning down, the sample was incubated for 5 min at room temperature. In a new DNA LoBind tube, 34 μL Sequencing Buffer (SQB), 25.5 μL Loading Beads (LB), 4.5 μL Nuclease-free water, and 11 μL DNA library were added. Meanwhile, for the priming of the flow cell FLO-MIN106 (R9.4 SpotON), 30 μL Flush Tether (FLT) was added directly to a tube of Flush Buffer (FB) and mixed by vortexing. 800 μL priming mix were loaded into the flow cell via priming port without introducing any air bubbles. After incubating for 5 min, the SpotON sample port was opened, and 200 μL priming mix were loaded into the flow cell via priming port. Finally, the prepared DNA library was mixed by pipetting up and down, and 75 μL of the sample volume was loaded into the flow cell via the SpotON sample port in a dropwise fashion. After closing the SpotON sample port and priming port and replacing the MinION lid, the sequencing experiment was started via MinKNOW software.

**Example 1**

There are several use cases for which nanopore adaptive sampling is beneficial. First, the depletion of known contaminants in a sample can increase the number of sequenced reads of interest. Second, targeting specific gene regions with adaptive sampling can increase the sequencing depth at those regions of interest to identify single-nucleotide variants and structural variations. Hence, in-silico enrichment of regions of interest can supersede the design of costly laboratory enrichment panels. However, the most exciting application probably is the sequencing of metagenomics samples, where enrichment of low-abundance genomes can reduce the time and costs required to answer a biological question (time-to-answer) and improve the ability to assemble these low copy genomes. This is especially interesting for pathogen detection in human or animal samples that suffer from up to 99.99% of sequenced reads being host background. For instance, Andrusch et al. 2018 reported only 45 unambiguously mapped reads to the Crimean-Congo hemorrhagic Fever Virus (CCHFV) genome out of 1,178,054 sequenced Illumina reads from a human serum sample.

In this example, the inventors explicitly focused on this last application of adaptive sampling in the exemplary context of nucleic acid analysis. That means the inventors aimed to deplete host background reads from human or animal samples to reduce the time to detect a pathogen and enrich the number of sequenced reads of that specific pathogen. Therefore, is was needed to query nanopore reads against large reference genomes. By design, adaptive sampling does not require to query complete nanopore reads against the reference but only the prefix part that is sequenced during the first few seconds after the DNA molecule entered the pore. This prefix is then classified as belonging to a region of interest or a depletion target region. Reads from regions of interest shall be sequenced as usual, while reads from depletion targets are rejected. In general, the faster and more accurately one can classify the reads, the faster one can reject uninteresting reads, which results in a shorter time-to-answer and higher enrichment of interesting regions. Thus, the classification step in conjunction with the base calling step needs to be fast enough to decide whether to eject a DNA molecule from the pore or continue sequencing as usual. Therefore, the inventors first evaluated the performance of the complete workflow described in section Workflow in an experiment where the goal was to deplete all human reads from a whole human genome sequencing run. The inventors used the playback feature offered by Oxford Nanopore's MinKNOW software to replay an already completed sequencing run for testing adaptive sampling. The inventors also compared the acquired results to those of Readfish (Payne, Holmes, Clarke, et al. 2021), which is, to the inventor's knowledge, the only tool that is fast enough to deplete nanopore reads of large reference sequences. This comparison reveals that the method according to the invention can make read rejection decisions in a smaller amount of time after the sequencing of a read has been started. As the inventors were not able to evaluate the classification performance with a playback run alone, they designed additional experiments that specifically focus on the evaluation of our read classification approach. Here it was assumed that the reads are already base called at this point in the workflow so that the present method was compared to minimap2 (Li 2018), which is used for classification by Readfish, and the recently published pan-genomics matching tool SPUMONI (Ahmed et al. 2021), which is proposed as an alternative to minimap2 in targeted nanopore sequencing pipelines. In the first two experiments, the inventors evaluated all three tools on simulated and real reads from a recently published microbial mock community (Nicholls et al. 2019). In a third experiment, the inventors used real nanopore reads from the human genome and a human gut microbiome and test the classification performance of all three tools.

The inventors also envision adaptive sampling as an application for pathogen detection in wildlife and domesticated animals. In such cases, researchers do not always have a reference genome of the host organism but can only use an available reference sequence of a closely related species to deplete the host reads during sequencing. For that reason, here nanopore reads were simulated from the *rattus norvegicus* reference genome and the read classification of the present method and minimap2 was evaluated by querying those reads against the reference genome of *mus musculus*.

Finally, the present example also shows the impact of read classification sensitivity and specificity on the attainable level of enrichment for low abundance genomic regions or organisms. For this purpose, the inventor expanded the mathematical model for potential enrichment by adaptive sampling proposed in a recent preprint by Martin et al. 2021. The sensitivity and specificity were integrated into this model and showed the importance of accurate classification for the potential enrichment.

Herein, all experiments for classification performance assessment were performed on a laptop with a 2.8 GHz Intel Core i7-7700HQ CPU and 16 GB of memory with an Ubuntu 20.04 OS installed. Each tool was run with a single thread for runtime comparisons. The recorded wall clock time and peak resident set size (RSS) were reported by the individual tools or GNU time 1.7.

**Example 2**

Live-Depletion Experiment

In the first Example of nucleic acid analysis herein, it was assessed whether the present read classification based on probabilistic data structures, e.g. here Interleaved Bloom filters, combined with DeepNano-blitz's CPU base calling is fast enough to keep up with the sequencing pace of the MinION. For this purpose, a bulk FAST5 file of a human whole-genome sequencing experiment was downloaded provided via the Github page of Readfish (https://github.com/LooseLab/readfish). Such a bulk FAST5 file (Payne, Holmes, Rakyan, et al. 2019) allows the playback of the whole sequencing run for testing if the ReadUntil functionality is working correctly. Oxford Nanopore's MinKNOW software simulates an already finished sequencing run without the need for a physical sequencing device when performing a play-back run. Compared to the original sequencing run, read signals are reported at the same time point after starting the run. During a playback run, unblocking a read does not cause MinKNOW to finish sending signals for that read. It just breaks the read at the time point of receiving an unblock message for the read and creates a new read identifier but continues to send signals of the same original read. To avoid inferences with MinKNOW running the playback script, it was decided to run the method according to the invention on a separate laptop with a remote connection to the MinKNOW control software. Since Readfish also needs a fast GPU for live base calling and it was desired to minimize delays caused by network transfer, Readfish was run on an NVIDIA Jetson AGX Xavier.

In the present experiment, a playback of a complete human genome sequencing run was performed with the goal to deplete all human reads from that run. This setup mimics the sequencing of a clinical human blood sample where up to 99% of the reads are human reads that the inventors aimed to deplete in order to enrich the number of reads from a pathogenic microbe. Playback runs were performed for 30 minutes on ONT's MinKNOW control software version 21.02.1. To ensure that the vast majority of the sequenced reads are of human origin, at first a playback run without adaptive sampling was performed. Reads were base called with Guppy 5.0.11 and mapped with minimap2 to the human Telomere-to-Telomere Consortium ("T2T") CHM13 v1.0 reference assembly (Miga et al. 2020). This resulted in 3274 reads passing the in-built quality filtering of MinKNOW, from which 99.66% could be mapped to the human reference sequence. This high proportion of human reads allows to compare the unblock performance of both tools by simply looking at the read lengths of unblocked reads. Since the goal here was to reject as many human reads as fast as possible, one aimed for a high number of unblocking decisions and short unblocked read lengths during the experiment.

For the comparison of the tools, first the *"break_reads_after_seconds"* parameter was adjusted within MinKNOW. For Readfish, the parameter were set to 0.4 seconds according to the author's recommendations. Since MinKNOW sends data as chunks, this parameter sets the size of one chunk to about 180 nucleotides. As the method according to the invention uses a minimum of 250 nucleotides for read classification, the inventors decided to set the parameter to 0.8 seconds so that the method according to the invention sees the first data chunk of a read after a maximum of 360 nucleotides have been sequenced. Both tools, the method according to the invention and Readfish, can concatenate the data chunks and perform classification after the receipt of every chunk. While here CPU base calling was used in the present method, the fast GPU mode version of the Guppy 5.0.11 base calling server was utilized for Readfish.

For the evaluation of both tools, the same playback run was repeated for 30 minutes. The inventors run the present approach with default parameters (*fragment_size* = 100, 000 and *kmer_size*= 13) using three base calling threads and three read classification threads, respectively. Using three threads is comparable to the chosen Readfish setting, where the base call server uses three threads and minimap2 using three threads per default. While the method according to the invention only uses the first five chunks of read data to classify reads and proceeds sequencing until the end if the read does not match, Readfish uses all read chunks and tries to classify the read until it is finished or rejected when running in depletion mode. The herein selected settings within the Readfish configuration file aim to unblock reads if they map to at least one human chromosome and proceed with sequencing if the read does not map. Thus, Readfish was evaluated with the *"max_chunks"* parameter set to "*inf*" and use the chunk log file to compute average and median read lengths at the point where an unblock decision was made. The same metrics were computed for the method according to the invention, which also reports unblocking decisions within a chunk log file. Table 1 shows the different length parameters for unblocked reads at the point of making an unblock decision and in the final output. It can be seen that both tools can reject most of the uninteresting reads after sequencing less than 450 nucleotides, and thus they can decide within one second of sequencing if a read shall be rejected or not. They report similar average read lengths, with Readfish having a larger standard deviation than the method according to the invention. The present tool also has a slightly smaller median read length than Readfish, suggesting that the method according to the invention can make unblock decisions a bit earlier in the sequencing process. Maximum and minimum read lengths reflect the parameter settings of both tools, with the method according to the invention only rejecting reads if at least 250 nucleotides and at most 1,800 nucleotides have been sequenced. Readfish has no lower or upper limit for read lengths when making unblock decisions, explaining the differing maximum and minimum read lengths. The maximum read length of Readfish also explains its higher standard deviation. However, the inventors argue that unblocks of such long reads are caused by alignment artifacts or low read quality and that the median read length is a better metric for assessing the unblock performance.

Next, all reads of both playback runs were base-called with Guppy 5.0.11 and the lengths of unblocked reads were investigated by analyzing the sequencing summary output files provided by MinKNOW and Guppy. The inventors observed that unblocked reads from the present method's playback run have shorter median and average read lengths than those from the Readfish playback run. This is also shown in the length distribution plots of unblocked reads for both playback runs presented in Figure 5. Surprisingly, the maximum unblocked read length in the final output of the playback run of the method according to the invention is 20,121 nucleotides, although an unblock decision for that same read was sent after 622 nucleotides were sequenced.

The unblocked reads acquired by the present method are on average 95 nucleotides shorter than those from the Readfish tool. This shorter read length correlates to about 0.2 seconds less time spent sequencing an uninteresting read when using the invention instead of Readfish, which can significantly impact the potential enrichment of low abundant genomes in a metagenomics sample. The read length distributions of unblocked reads are depicted in Table 1 and Figure 5.

**Table 1:** Comparison of lengths from reads unblocked by the present method and Readfish on a 30 minutes playback of a whole human sequencing run. Read lengths are compared at the time point when the respective tool made the decision (reported via log files) and in the final output after the playback run has been finished.

| | Point of decision | | Final output | |
|---|---|---|---|---|
| Tool | Invention | Readfish | Invention | Readfish |
| Average read length | 383.36 | 382.38 | 625.40 | 719.32 |
| Standard deviation | 135.66 | 656.19 | 669.62 | 649.40 |
| Median read length | 331 | 348 | 491 | 686 |
| Max read length Min | 1,477 | 92,063 | 20,121 | 92,760 |
| read length | 250 | 62 | 184 | 80 |

Evaluating Read Classification

Experimental Setup:

During a nanopore targeted sequencing experiment with ONT's ReadUntil functionality, the sequencing device transmits electrical current data via the MinKNOW control software to the method according to the invention. This data is received as chunks, representing a maximum of 0.4 or 0.8 seconds of sequencing, depending on the MinKNOW configuration. Since a DNA molecule translocates through the pore at a speed of about 450 bases per second, 0.4 seconds of sequencing represent about 180 bases of data. In the following experiments, the situation was mimicked where a chunk represents 0.8 seconds of sequencing data received and base called immediately by the method according to the invention. This time interval was chosen for the following practical considerations. Using 360 nucleotides guarantees that base calling on a CPU is fast enough to keep up with the sequencing pace, and the inventors further wanted to make rejection decisions as early as possible while still being able to classify most of the reads correctly. In this section, it was assumed that base-calling has already been performed. For a fair comparison, all experiments were set up in such a way that all three tools, minimap2, SPUMONI, and the method according to the invention, attempt to classify reads based on the 360 bases long read prefix. In practice, all reads, both simulated and real reads, were cut to only the first 360 bases. The method according to the invention then hashes all k-mers of these 360 bases and compares the hash values to a prebuild probabilistic data structure, e.g. an interleaved Bloom filter, of the depletion target references to make classification decisions.

The software's default settings were used for the SPUMONI approach, which means splitting the prefix into substrings of 90 nucleotides each for further read classification. SPUMONI also needs a prebuild index of the references but has to include the reverse complement of the depletion target references. SPUMONI matches the substrings against this positive index and a null index, consisting of the reverse sequences of the positive index. Finally, classification decisions are made by using a Kolmogorov-Smirnov test.

For the minimap2-based approach, the read classification of Readfish was mimicked by using the *mappy* Python interface (https://pypi.org/project/mappy/) for minimap2. For comparison, the read prefixes were aligned with the *map-ont* settings without applying any mapping quality filter, which are the same settings used by Readfish. To evaluate the three tools, reads correctly classified as belonging to the depletion target are considered true positives (TP), while reads falsely classified for depletion are called false positives (FP). Consistently, reads that are correctly not classified as depletion target are considered true negatives (TN), and reads belonging to the depletion target but not classified for depletion are called false negatives (FN). The classification accuracy was calculated together with the precision, recall, specificity, and F1-score for all three approaches based on those considerations. Since an imbalanced number of sequenced reads between depletion and enrichment targets was assumed, the Matthews correlation coefficient (MCC) in every experiment was also reported.

Simulated Mock Community

In the first dataset, simulated ONT-like reads were considered that were derived from the identical genomes of the ZymoBIOMICS High Molecular Weight DNA Mock Microbial community (ZymoMC) (Nicholls et al. 2019). This mock community consists of seven bacterial species - *Enterococcus faecalis, Listeria monocytogenes, Bacillus subtilis, Salmonella enterica, Escherichia coli, Staphylococcus aureus,* and *Pseudomonas aeruginosa* - as well as *Saccharomyces cerevisiae.* PBSIM2 (Ono et al. 2021) was used to simulate Oxford-Nanopore-like reads (R9.4 pores) from Zymo Mock Community references at varying levels of mean read accuracy: 80%, 85%, 90%, 95%, and 98%. Furthermore, proportions of reads from each genome were simulated in such a way to mimic a scenario with few Saccharomyces cerevisiae reads (Figure 6). The goal here is to enrich *Sac-charomyces cerevisiae* sequences by correctly classifying bacterial reads, which one would aim to eject from the pores in a real nanopore sequencing run. Therefore, an index of the seven bacterial reference genomes was build and query all bacterial and yeast reads against the index. Consistent with our definition in section Experimental Setup, correctly classified bacterial reads were considered true positives (TP), while yeast reads found in the index are considered false positives (FP). In addition, bacterial reads that are missed to be found by a tool in the index as false negatives (FN) were defined, and yeast reads that are not found in the index are considered true negatives (TN).

As shown in Table 2, accuracy, as well as the other related measures, improve with increasing read accuracy for all three tools. On all read accuracy levels, the present method demonstrates slightly but consistently better accuracy, recall, F1-scores, and MCC than SPUMONI, while both tools outperform minimap2. The precision of all three tools is very high, with the present method and minimap2 both having the highest scores on all read accuracy levels. Minimap2 is also the only tool that shows 100% specificity, but the present method comes close to 100% as well. SPUMONI lags a bit behind the specificity scores of the other two tools. Figure7 visualizes recall and specificity for the three tools across the various read accuracies. It can be seen that the method according to the invention is the best performing tool for this read classification task. It combines high recall (sensitivity) with high specificity. The other two prior art tools either have high recall but lower specificity or high specificity but lower recall scores.

**Table 2:** Comparing the present method, SPUMONI, and minimap2 across various metrics on a simulated Zymo Mock Community consisting of seven bacterial species and Saccharomyces cerevisiae. We simulated **360** nucleotide long reads of varying levels of sequence accuracy for all eight organisms. All reads were mapped against the seven bacterial reference sequences to filter out only the bacterial reads. At the same time, one would want to keep as much Saccharomyces cerevisiae reads, which corresponds to an enrichment of that organism in a real-world experiment. The method according to the invention can classify a higher percentage of bacterial reads at all levels of read accuracy while having only slightly less specificity than minimap2.

| Read Accuracy (%) | 80 | | | 85 | | | 90 | | |
|---|---|---|---|---|---|---|---|---|---|
| **Tool** | Invention | SPUMONI | minimap2 | Invention | SPUMONI | minimap2 | Invention | SPUMONI | minimap2 |
| Accuracy | **69.16** | 68.23 | 62.03 | **89.83** | 89.15 | 86.19 | **96.74** | 96.43 | 94.48 |
| Precision | **100.00** | 99.88 | **100.00** | **100.00** | 99.90 | **100.00** | **100.00** | 99.91 | 100.00 |
| Recall | **68.48** | 67.61 | 61.19 | **89.61** | 89.00 | 85.89 | **96.67** | 96.44 | 94.36 |
| Specificity | 99.98 | 96.32 | **100.00** | 99.95 | 95.99 | **100.00** | 99.94 | 96.01 | 100.00 |
| F1-Score | **82.29** | 80.64 | 75.92 | **94.51** | 94.13 | 92.41 | **98.31** | 98.14 | 97.10 |
| MCC | **0.21** | 0.20 | 0.18 | **0.40** | 0.37 | 0.34 | **0.62** | 0.59 | 0.52 |
| **Read Accuracy (%)** | 95 | | | 98 | | | | | |
| **Tool** | Invention | SPUMONI | minimap2 | Invention | SPUMONI | minimap2 | | | |
| Accuracy | **99.00** | 98.84 | 97.19 | **99.28** | 99.13 | 97.52 | | | |
| Precision | **100.00** | 99.91 | **100.00** | **100.00** | 99.91 | **100.00** | | | |
| Recall | **98.98** | 98.90 | 97.13 | **99.27** | 99.20 | 97.47 | | | |
| Specificity | 99.93 | 95.90 | **100.00** | 99.88 | 95.98 | **100.00** | | | |
| F1-Score | **99.49** | 99.40 | 98.54 | **99.63** | 99.56 | 98.72 | | | |
| MCC | **0.82** | 0.79 | 0.65 | **0.86** | 0.83 | 0.67 | | | |

Real Mock Community

Next, the present method was applied to real nanopore reads from a Zymo Mock Community. After sample preparation, the mock sample was sequenced for 48 hours on a MinION flowcell (no. FLO-MIN106) with v.R9.4.1 pores. We used this run to test our ReadUntil client software and unblocked every second read on odd-numbered channels, which resulted in a lower sequencing yield. Obtained Fast5 files were base called with DeepNano-blitz using a recurrent neural network size of 48. For better comparison with minimap2, we first build a separately obtained minimap2 mapping as a gold standard. Therefore, all reads shorter than 2,000 base pairs were filtered out and the first 360 nucleotides from each read were trimmed since we use these bases for later classification. Then, the trimmed reads were mapped with standard ONT settings to the ZymoMC reference genomes and only reads with a mapping quality score bigger or equal to 30 are considered confidently mapped. From these mapped reads, the trimmed 360 nucleotide long prefixes are used for the read classification by the three tools again. Figure 8 shows the resulting proportions of reads from each genome.

In this experiment, we also measure the peak Resident Set Size (RSS) and index size in GigaByte and the throughput for each of the tools in reads classified per second.

Results in Table 3 show that the present method achieves better accuracy, recall, and F1-score than SPUMONI and minimap2, which both have similar results for those three measures. Minimap2 has slightly better precision and specificity than the method according to the invention. While SPUMONI has almost the same precision as the present method and minimap2, it shows significantly less specificity. These results are consistent with those for the simulated data sets in section Live-Depletion Experiment and show that the method according to the invention outperforms the other two tools on read classification for short nanopore reads.

Another important aspect is the amount of main memory a tool needs to hold the reference index needed for read classification. Using the seven bacterial reference genomes of the Zymo Mock Community as depletion target (reference index), the method according to the invention shows the smallest maximum memory consumption measured as Peak Resident Set Size (RSS). It only needs 0.099 GigaBytes (GB) of main memory, in contrast to 0.272 GB consumed by minimap2. Furthermore, the present method has the smallest index file size (0.047 GB) of all three tools. In addition to the smallest memory footprint, the method according to the invention also achieves the highest classification throughput. We can classify 5967 reads per second with our approach compared to 5632 reads per second by minimap2 and 1102 reads per second achieved by SPUMONI. These results show that the method according to the invention correctly classifies more reads than the other tools. and is also computationally more efficient than other state-of-the-art read classification tools used for nanopore adaptive sampling.

Table 3: Comparing the method according to the invention, SPUMONI, and minimap2 across various metrics on a real Zymo Mock Community data set consisting of seven bacterial species and *Saccharomyces cerevisiae*. Reads from a nanopore sequencing run are mapped to the eight organisms to generate ground truth. Only the first 360 nucleotides were used for classification from those confidently mapped reads to mimic unblock decision-making after 0.8 seconds of sequencing the individual read. All reads are mapped against the seven bacterial reference sequences to filter out only the bacterial reads. At the same time, we want to keep as much *Saccharomyces cerevisiae* reads, which corresponds to an enrichment of that organism in an enrichment/depletion experiment. Consistent with the simulated data, the method according to the invention can classify a higher percentage of bacterial reads while having only slightly less precision and specificity than minimap2. The present approach also shows to be the computationally most effective one, with the lowest memory footprint and highest classification throughput.

| Tool | Invention | SPUMONI | minimap2 |
|---|---|---|---|
| Accuracy | **94.50** | 90.96 | 89.33 |
| Precision | 99.99 | 99.89 | **100.00** |
| Recall | **94.38** | 90.85 | 89.08 |
| Specificity | 99.73 | 95.87 | **99.95** |
| F1-Score | **97.10** | 95.16 | 94.23 |
| MCC | **0.52** | 0.41 | 0.39 |
| Peak RSS (GB) | **0.099** | 0.163 | 0.272 |
| Index Size (GB) | **0.047** | 0.153 | 0.097 |
| Throughput (reads per sec) | **5967** | 1102 | 5632 |

**Example 3**

Real Human Gut Microbiome

In a third classification Example, the inventors tested the present method on a more advanced scenario where the goal was to deplete all human host reads from a human gut microbiome sample to enrich the microbial species in that sample. This is a typical use case for nanopore adaptive sampling, where researchers want to study a microbiome but will also have a lot of human DNA in their samples, which is not interesting to them. Here, the inventors aimed for an in-silico depletion of human reads to achieve enrichment of low abundance organisms of the microbiome that they would not see otherwise. Herein such a dataset was constructed by mixing real nanopore reads from a human gut microbiome study (Moss et al. 2020) with real nanopore reads from sequencing of Ashkenazi son cell line HG002 performed as part of the Genome-In-A-Bottle consortium (Zook et al. 2020). Human reads were already filtered out from the microbiome dataset so that the only human reads in our constructed dataset originate from the Ashkenazi son cell line sequencing. Having filtered out human reads from the microbiome dataset ensures a ground truth in our mixed dataset that lets us reliably calculate true positives, true negatives, false positives, and false negatives. Based on those values, the inventors assessed the classification performance using the same standard metrics as in the sections above. Since the goal was to classify reads as fast as possible for rejection, the invention was set to take again only the first 360 nucleotides of the reads to classify them as being of human origin or not. As in the previous experiments, this mimics the number of nucleotides sequenced within 0.8 seconds after sequencing of a particular read has begun. First the corresponding index files for all three tools under investigation were built: The present method, minimap2, and SPUMONI. Indexes are built by using the Telomere-to-Telomere Consortium ("T2T") CHM13 v1.0 assembly (Miga et al. 2020; Nurk et al. 2021). The inventors evaluated the classification performance of the three tools by querying the 360 nucleotide prefixes of all reads against their corresponding indexes. While using small genomes in a reference index is a trivial task, the challenge for all three tools in this setting is the larger reference sequence, requiring more memory consumption and more time to classify a read.

When looking at the performance metrics provided in Table 4, one can see results similar to those from the Zymo Mock Community experiments, with the method according to the invention showing significantly better accuracy, recall, MCC, and F1-Score compared to minimap2 and SPUMONI. Although minimap2 shows better precision and specificity, a tiny difference between the inventor's approach and minimap2 can be observed for those metrics. The results in Table 4 also demonstrate that the present method has a much smaller memory footprint and creates smaller index files than the other tools. However, it is recognizable that the method according to the invention has the smallest read classification throughput of all three tools. Regarding this metric, minimap2 outperforms the present method and SPUMONI. The drastic decrease in classification speed by the present method is explainable by a current limitation of the probabilistic data structure , e.g. interleaved Bloom filter, implementation, which leads to a large number of bins resulting from the fragmentation step. As described in the methods section, this fragmentation step is required to classify error-prone nanopore reads reliably. Although a throughput of 172 reads per second may appear too small to keep up with the parallel sequencing of up to 512 nanopores, it does not cause any problems in practice when using three classification threads. Internal measurements of the average read classification time showed no difference to the average base calling time of DeepNano-blitz.

Furthermore, Payne, Holmes, Clarke, et al. 2021 described in their publication that the GPU version of the Guppy base caller needed on average 0.28 seconds to call an average batch of 30 reads. This corresponds to roughly 107 reads per second, which is even slower than our read classification. Thus, we do not expect our classification speed to cause a bottleneck in the overall process.

**Table 4.** Comparing the present method, SPUMONI, and minimap2 across various metrics on a real Human gut micro-biome data set. We mix nanopore reads from a whole human sequencing run with human gut microbiome reads. We mapped the first 360 nucleotides of all reads against the human reference sequence, which mimics unblock decision-making after 0.8 seconds of sequencing the individual read. Here the goal is to deplete human reads from the mixed sample while keeping as many microbiome reads as possible to enrich low abundant organisms in that sample. The present method enables the classification of a higher percentage of human reads while having only slightly less precision and specificity than minimap2. Our approach also has the lowest memory footprint and smallest index size. In contrast, the present method shows the smallest throughput of all three tools.

| Tool | Invention | minimap2 | SPUMONI |
|---|---|---|---|
| Accuracy | 97.84 | 96.53 | 93.08 |
| Precision | 98.86 | 99.99 | 81.11 |
| Recall | 89.20 | 80.98 | 80.91 |

(continued)

| Tool | Invention | minimap2 | SPUMONI |
|---|---|---|---|
| Specificity | 99.77 | 99.99 | 95.80 |
| F1-Score | 93.78 | 89.49 | 81.01 |
| MCC | 0.93 | 0.83 | 0.77 |
| Peak RSS (GB) | 8.83 | 11.643 | 14.69 |
| Index Size (GB) | 4.50 | 6.96 | 16.4 |
| Throughput (reads per sec) | 172 | 1993 | 598 |

Classification without reference of depletion target

When investigating animal infectious diseases, there are cases where researchers take metagenomics samples from an animal where no reference sequence of the host is available. In such cases, the researchers will use the reference sequence of the closest related species available to facilitate the depletion of host nanopore reads. Although not perfect, even the depletion of only 25% of host nanopore reads could enrich the pathogen reads to a sufficient level for further analysis without laboratory expensive enrichment methods. This section exemplifies the calculation of the potential enrichment of a low abundant organism. Setting the sensitivity in the calculations to 25%, means that 25% of the host reads could correctly be unblocked, which yields an enrichment factor of 1.27. If one also assumes that in an experiment without adaptive sampling, only 15 reads of a pathogenic organism would be sequenced, even this low sensitivity can lead to four more reads being sequenced with adaptive sampling. These four additional reads can have a massive impact if they span antibiotic resistance genes.

To mimic such an experimental setting, the reference sequences of two closely related species, *rattus norvegicus* (GCF_015227675.2) and *Mus musculus* (GCF_000001635.27), from the NCBI RefSeq database were used. First, nanopore-like reads from the rat genome with a mean accuracy of 90% were simulated using PBSIM2 (Ono et al. 2021) and mixed with the simulated Zymo Mock Community reads from section Simulated Mock Community, which had a mean accuracy of 90% as well. Then, an index of the mouse reference genome was created and the 360 nucleotide long prefixes of all reads from this metagenomics sample were mapped against the mouse index. Herein, the aim was to classify and deplete as many *rattus norvegicus* reads as possible from this sample using the *Mus musculus* reference genome while keeping as many microbial reads for sequencing as possible.

The inventors evaluated the classification performance of the method according to the invention and minimap2 for this scenario by using the same statistical metrics as in the section above. Here, the results of the present method and minimap2 were not compared with SPUMONI because a SPUMONI index for the *Mus musculus* reference genome could not be built on several operating systems with the indexing step terminating with an error message. Since SPUMONI is in none of the experiments the best performing tool on any metric, it is assumed that this will not influence the informative value of this experiment.

**Table 5.** Comparing the present method and minimap2 across various metrics on a mixture of simulated microbial and simulated rat reads. Here, we mix simulated nanopore reads from the *rattus norvegicus* reference with simulated Zymo Mock Community reads. We map the first 360 nucleotides of all reads against the *Mus musculus* reference sequence, which mimics unblock decision-making after 0.8 seconds of sequencing the individual read. This experiment aims to deplete *rattus norvegicus* reads from the mixed sample while keeping as many microbial reads as possible. This depletion experiment mimics a use case where no reference of the host is available. present method can classify a higher percentage of *rattus norvegicus* reads while having only slightly less precision and specificity than minimap2. The method according to the invention also has the lowest memory footprint and smallest index size. Minimap2 shows significantly higher throughput than present method.

| Tool | Invention | minimap2 |
|---|---|---|
| Accuracy | 43.26 | 27.30 |
| Precision | 99.99 | 100.00 |
| Recall | 36.83 | 19.07 |
| Specificity | 99.97 | 100.00 |
| F1-Score | 53.84 | 32.04 |
| MCC | 0.24 | 0.15 |
| Peak RSS (GB) | 6.98 | 13.94 |
| Index Size (GB) | 4.18 | 6.64 |

(continued)

| Tool | Invention | minimap2 |
|---|---|---|
| Throughput (reads per sec) | 171 | 6448 |

The results provided in Table 5 are consistent with those of the human microbiome experiment. Again, the method according to the invention shows better accuracy, recall, F1-Score, and MCC than minimap2, which has slightly higher precision and specificity than the present method. The inventors also measured a smaller memory footprint and smaller index file size for the present method while minimap2 has significantly higher throughput. Astonishingly, one can see a big difference of 18 percent at recall and 13.5 percent accuracy between the two tools. Thus, the method according to the invention is much more suitable than Readfish for host read depletion by using the reference sequence of closely related organisms.

Potential Enrichment of low abundance organisms

One of the key promises of adaptive sampling is the potential for in-silico enrichment of low abundance organisms in metagenomics samples. A typical use case is the pathogen detection in a clinical human blood sample, where only about 1% of sequenced reads originate from the pathogen organism. In order to reliably detect the pathogen in such a metagenomics sample, different laborious approaches are available to enrich the pathogen DNA. However, these approaches require sample-specific adaptation and thus are not universally applicable. In their recent preprint, Martin et al. showed that adaptive sampling might enrich low abundance organisms depending on different experimental parameters like average read length, expected a priori abundance of the organisms to enrich, and the response time to unblock unwanted DNA sequences. Here we expand their mathematical model by incorporating the classification sensitivity and specificity into their formula for enrichment to show the effect of different classification accuracies on the enrichment potential.

In general, their model calculates the enrichment factor $e_x$ for an organism $x$ as the quotient of $T_e$, the proportion of sequencing time spent sequencing reads belonging to $x$ with adaptive sampling, and $T$, the proportion of sequencing time spent sequencing reads belonging to x without adaptive sampling.

$$e_x = \frac{T_e}{T} \qquad (1)$$

Further, $R$ is defined as the average read size and $C$ as the time taken for a pore to capture a new molecule after sequencing the last molecule. Assuming a constant sequencing rate $S$ in units of number of bases sequenced per second, one can define the average sequencing time for a molecule as $R/S$. With $x_{ab}$ being the proportion of molecules aimed to enrich, one gets

$$T = \frac{x_{ab}(R/S)}{R/S + C} \qquad (2)$$

For the calculation of $T_e$, one defines D as the average length of depleted reads, so that $D/S$ is the average time spent sequencing a molecule classified for depletion. And so the total sequencing time for an experiment with adaptive sampling is given by $x_{ab}(R/S) + (1 - x_{ab})(D/S) + C$, and so

$$T_e = \frac{x_{ab}(R/S)}{x_{ab}(R/S) + (1 - x_{ab})(D/S) + C} \qquad (3)$$

This mathematical enrichment model has the disadvantage of assuming perfect classification accuracy with 100% sensitivity and 100% specificity. However, as shown in section *Evaluating Read Classification,* sensitivity and specificity will rarely reach 100%, both simultaneously. Therefore, the inventors added the classification sensitivity K and specificity M to equation (3) and modified the nominator to $x_{ab} \times K \times (R/S)$, which is the time spent sequencing molecules not classified for depletion. In this sense, one can split the denominator into four terms that, in addition to the (1) nominator, also represent the (2) time spent sequencing molecules falsely classified for depletion, (3) time spent sequencing molecules correctly classified for depletion and the (4) time spent sequencing molecules missed to deplete.

**Table 6**. Comparing the enrichment potential of Readfish and the present method based on their read classification performance for different library sizes. The average length of depleted reads comes from the observations in the Live-Depletion experiment in section *Live-Depletion Experiment.* Specificity and sensitivity values stem from the evaluation of the present method and minimap2 on the human microbiome dataset in section *Real Human Gut Microbiome.* Library sizes correspond to the expected average read length when sequencing such a sample. Thus, the present method shows a higher potential for enrichment of low abundant organisms on all library sizes.

|  | Readfish (minimap2) | Invention |
|---|---|---|
| **Average length of depleted** | 719 | 625 |
| **Specificity M** | 99.99 | 99.77 |
| **Sensitivity K** | 80.98 | 89.20 |
| **Enrichment factor ex for 2,5 kb** | 2.12 | 2.58 |
| **Enrichment factor ex for 5 kb** | 2.97 | 3.94 |
| **Enrichment factor ex for 7,5 kb** | 3.46 | 4.84 |
| **Enrichment factor ex for 10 kb** | 3.77 | 5.47 |
| **Enrichment factor ex for 15 kb** | 4.15 | 6.31 |

This gives us

$$T_e = \frac{x_{ab}K(R/S)}{x_{ab}K(R/S) + x_{ab}(1-K)(D/S) + (1-x_{ab})M(D/S) + (1-x_{ab})(1-M)(R/S) + C} \quad (4)$$

Using this equation, one can evaluate the impact of the different classification tools on the potential enrichment. Therefore, the inventors took the example from above and assumed a metagenomics sample with 99.99% human sequenced reads they would like to deplete in order to enrich organisms in the residual 0.01% ($x_{ab}$= 0.0001). It is further assumed that the sequencing speed $S$ = 450 bases per second and a capturing time $C$ = 0.5 seconds. Table 6 shows the difference in enrichment potential of Readfish and the present method for different library sizes sequenced on a MinION. Here the measured average read length of depleted human reads in the final output from section Live-Depletion Experiment was used. The results show an average length of depleted reads of 719 nucleotides for Readfish and 625 nucleotides for the present method. Herein, the values for sensitivity (recall) and specificity from the human microbiome experiment in section *Real Human Gut Microbiome* were used, assuming a specificity of 99.99 for Readfish and 99.77 for the present method as well as a sensitivity of 80.98 for Readfish and 89.20 for the present method. As already shown by Martin et al., the potential for enrichment increases with the library size. That means the longer the DNA fragments in the sample, the more benefit is expected from the application of adaptive sampling. Because the longer an uninteresting fragment is, the more sequencing time can be saved for interesting fragments when rejecting these long uninteresting DNA molecules from the pores. Additionally, one can see that classification performance influences the potential for enrichment as well. As shown in Table 6, the present method's higher sensitivity results in higher enrichment of low abundance organisms in a metagenomics sample. For example, when sequencing a library with an insert size of 15 kilobases (which should theoretically result in an average sequenced read length of 15,000 nucleotides), Readfish would only reach an enrichment factor of 4.15, while the present method could enrich the low abundance proportion of reads by a factor of 6.31. In an experiment with about 400,000 sequenced reads, Readfish would increase the number of reads from low abundance organisms from 40 to 166. For the same experiment, the method according to the invention could increase the number of sequenced reads from 40 to 252. This difference of 50% more reads after adaptive sampling can improve downstream bioinformatics analysis massively. If, for instance, the low abundant organism researchers aim to enrich is a pathogen with a genome size of roughly 2.5 Megabases, the 40 reads from the original experiment without adaptive sampling would only cover 600,000 sequenced bases of the pathogen. In this case, less than half of the complete genome of that pathogen would be sequenced. Using Readfish with an enrichment factor of 4.15, roughly 2.49 Megabases of the pathogen could be sequenced, which is still insufficient to cover the whole pathogen genome. With the method according to the invention, the number of sequenced nucleotides from the pathogen can be increased to 3.78 Megabases, theoretically covering each position in the pathogen genome at least once. From this example, one can see that an improved classification that leads to increased enrichment can improve genome assembly of low abundant organisms and increase the chance to sequence antibiotic resistance genes, which we would not detect without adaptive sampling.

**References**

Ahmed O, Rossi M, Kovaka S, Schatz MC, Gagie T, Boucher C, and Langmead B. 2021. Pan-genomic Match-ing Statistics for Targeted Nanopore Sequencing. iScience. 102696.

Andrusch A, Dabrowski PW, Klenner J, Tausch SH, Kohl C, Osman AA, Renard BY, and Nitsche A. 2018. PAIPline: pathogen identification in metagenomic and clinical next generation sequencing samples. Bioinformatics. 34: i715-i721.

Blanca A, Harris RS, Koslicki D, and Medvedev P. 2021. The statistics of k-mers from a sequence undergoing a simple mutation process without spurious matches. bioRxiv.

Bloom BH. 1970. Space/time trade-offs in hash coding with allowable errors. Communications of the ACM. 13: 422-426.

Boža V, Peresı̌ni P, Brejová B, and Vinař T. 2020. DeepNano-blitz: a fast base caller for MiniON nanopore sequencers. Bioinformatics. 36: 4191-4192.

Brinkerhoff, H. (1), Kang, A. S. W., Liu, J., Aksimentiev, A., Dekker, C.; Code and Data for "Multiple rereads of single proteins at single-amino acid resolution using nanopores", Science; 4 Nov 2021; Vol 374, Issue 6574; pp. 1509-1513; DOI: 10.1126/science.abl4381

Brinkerhoff, H. (2), Kang, A.S.W., Liu, J., Aksimentiev, A., Dekker, C., 2021, bioRxiv 2021.07.13.452225; "Infinite re-reading of single proteins at single-amino-acid resolution using nanopore sequencing", doi: https://doi.org/10.1101/2021.07.13.452225

Broder AZ 1997. On the resemblance and containment of documents. In: Proceedings. Compression and Complexity of SEQUENCES 1997 (Cat. No. 97TB100171). IEEE, pp. 21-29.

Dadi TH, Siragusa E, Piro VC, Andrusch A, Seiler E, Renard BY, and Reinert K. 2018. DREAM-Yara: An exact read mapper for very large databases with short update time. Bioinformatics. 34: i766-i772.

Edwards HS, Krishnakumar R, Sinha A, Bird SW, Patel KD, and Bartsch MS. 2019. Real-time selective sequencing with RUBRIC: read until with basecall and reference-informed criteria. Scientific reports. 9: 1-11.

Kietbasa SM, Wan R, Sato K, Horton P, and Frith MC. 2011. Adaptive seeds tame genomic sequence comparison. Genome research. 21: 487-493.

Kono, Nobuaki & Arakawa, Kazuharu. (2019). Nanopore sequencing: Review of potential applications in functional genomics. Development, Growth & Differentiation. 61. 10.1111/dgd.12608.

Koslicki D and Zabeti H. 2019. Improving minhash via the containment index with applications to metage-nomic analysis. Applied Mathematics and Computation. 354: 206-215.

Kovaka S, Fan Y, Ni B, Timp W, and Schatz MC. 2021. Targeted nanopore sequencing by real-time mapping of raw electrical signal with UNCALLED. Nature Biotechnology. 39: 431-441.

Leggett RM and Clark MD. 2017. A world of opportunities with nanopore sequencing. Journal of Experi-mental Botany. 68: 5419-5429.

Li H. 2018. Minimap2: pairwise alignment for nucleotide sequences. Bioinformatics. 34: 3094-3100.

Loose M, Malla S, and Stout M. 2016. Real-time selective sequencing using nanopore technology. Nature methods. 13: 751-754.

Martin S, Heavens D, Lan Y, Horsfield S, Clark MD, and Leggett RM. 2021. Nanopore adaptive sampling: a tool for enrichment of low abundance species in metagenomic samples. bioRxiv.

Miga KH, Koren S, Rhie A, Vollger MR, Gershman A, Bzikadze A, Brooks S, Howe E, Porubsky D, Logsdon GA, et al. 2020. Telomere-to-telomere assembly of a complete human X chromosome. Nature. 585: 79- 84.

Mikheyev AS and Tin MM. 2014. A first look at the Oxford Nanopore MiniON sequencer. Molecular ecology resources. 14: 1097-1102.

Mongan AE, Tuda JSB, and Runtuwene LR. 2020. Portable sequencer in the fight against infectious disease. Journal of human genetics. 65: 35-40.

Moss EL, Maghini DG, and Bhatt AS. 2020. Complete, closed bacterial genomes from microbiomes using nanopore sequencing. Nature biotechnology. 38: 701-707.

Andreas D. Baxevanis, Gary D. Bader, David S. Wishart, 2020, Bioinformatics, Wiley-Blackwell, 4th Edition.

Nicholls SM, Quick JC, Tang S, and Loman NJ. 2019. Ultra-deep, long-read nanopore sequencing of mock microbial community standards. Gigascience. 8: giz043.

Nurk S et al. 2021. The complete sequence of a human genome. bioRxiv.

Ondov BD, Treangen TJ, Melsted P, Mallonee AB, Bergman NH, Koren S, and Phillippy AM. 2016. Mash:fast genome and metagenome distance estimation using MinHash. Genome biology. 17: 1-14.

Ono Y, Asai K, and Hamada M. 2021. PBSIM2: a simulator for long-read sequencers with a novel generative model of quality scores. Bioinformatics. 37: 589-595.

Oxford Nanopore Technologies 2020. Nanopore Sequencing Accuracy. https://nanoporetech.com/ accuracy. [Online; accessed 23-June-2021].

Oxford Nanopore Technologies 2021. Minknow API. Version 4.2.4.

Payne A, Holmes N, Clarke T, Munro R, Debebe BJ, and Loose M. 2021. Readfish enables targeted nanopore sequencing of gigabase-sized genomes. Nature biotechnology. 39: 442-450.

Payne A, Holmes N, Rakyan V, and Loose M. 2019. BulkVis: a graphical viewer for Oxford nanopore bulk FAST5 files. Bioinformatics. 35: 2193-2198.

Piro VC, Dadi TH, Seiler E, Reinert K, and Renard BY. 2020. ganon: precise metagenomics classification against large and up-to-date sets of reference sequences. Bioinformatics. 36: i12-i20.

Quick J, Loman NJ, Duraffour S, Simpson JT, Severi E, Cowley L, Bore JA, Koundouno R, Dudas G, Mikhail A, et al. 2016. Real-time, portable genome sequencing for Ebola surveillance. Nature. 530: 228-232.

Rang FJ, Kloosterman WP, and Ridder J de. 2018. From squiggle to basepair: computational approaches for improving nanopore sequencing read accuracy. Genome biology. 19: 1-11.

Runtuwene LR, Tuda JS, Mongan AE, and Suzuki Y. 2019. On-site MiniON sequencing. Single Molecule and Single Cell Sequencing. 143-150.

Sim J and Chapman B. 2019. In-field whole genome sequencing using the MiniON nanopore sequencer to detect the presence of high-prized military targets. Australian Journal of Forensic Sciences. 51: S86-S90.

Wick RR, Judd LM, and Holt KE. 2019. Performance of neural network base calling tools for Oxford Nanopore sequencing. Genome biology. 20: 1-10.

Zook JM, Hansen NF, Olson ND, Chapman L, Mullikin JC, Xiao C, Sherry S, Koren S, Phillippy AM, Boutros PC, et al. 2020. A robust benchmark for detection of germline large deletions and insertions. Nature biotechnology. 1-9.

**Claims**

1. Method for determining the presence of one or more target sequences in a biopolymer-comprising sample, comprising:

   providing a nanopore sequencing library generated from the biopolymer-comprising sample,
   subjecting the sequencing library to a nanopore sequencing process comprising the following analysis steps:

   i. obtaining a biopolymer sequence by sequencing 10-1500 consecutive building blocks of a biopolymer fragment in a nanopore of a nanopore sequencing device,
   ii. comparing the biopolymer sequence obtained in step i. to sequences comprised within a decoy sequence database and/or comparing the biopolymer sequence obtained in step i. to the one or more target sequences comprised within a target sequence database by applying a probabilistic data structure approach that utilizes hash functions, subsequences and bit arrays for sequence comparison,
   wherein

   a. if the sequence obtained in step i. matches to at least one decoy sequence comprised by the decoy sequence database but not to the sequences comprised by the target database, the sequencing of the respective biopolymer fragment is aborted and the biopolymer fragment is discarded from the nanopore, or
   b. if the sequence obtained in step i. matches to at least one target sequence comprised by the target sequence database but not to the sequences comprised by the decoy sequence database the presence of the target sequence of the target sequence database with the highest shared sequence identity to the obtained sequence is determined, and preferably the sequencing of the biopolymer fragment is continued, preferably until the end of the biopolymer fragment is reached and a final sequence is obtained, or
   c. if the sequence obtained in step i. neither matches to at least one sequence comprised by the decoy sequence database nor to at least one sequence comprised by the target sequence database, or

   if the sequence obtained in step i. matches to at least one sequence comprised by both the decoy sequence database and the target sequence database,
   the sequencing of the biopolymer fragment in the nanopore of step i. is continued for a defined length of building blocks, preferably at least 100 building blocks, or alternatively until the end of the fragment, resulting in an elongated sequence,
   wherein the obtained elongated sequence is compared to sequences comprised within the decoy sequence database and/or to sequences comprised within the target sequence database according to step ii., wherein one of alternative steps ii. a., b. or c. is performed,

   iii. optionally determining the presence of one or more target sequences in the sample according to step ii.b.

2. Method according to claim 1, wherein the decoy and/or the target sequence database are dynamically indexable databases, which can be modified during any of the method steps i. to iii, wherein the decoy and/or the target sequence database are preferably modified according to the target sequences determined under step ii b.

3. Method according to claim 2, wherein the comparing in step ii. comprises applying a 95%-confidence interval for the number of matching subsequences between the biopolymer fragment sequenced in step i. and a sequence from the decoy and/or the target sequence database.

4. Method according to any one of the preceding claims, wherein the probabilistic data structure is an interleaved bloom filter (IBF), an interleaved XOR filter (IXF) or an interleaved binary fuse filter (IFF).

5. Method according to any one of the preceding claims, wherein step ii. is repeated if the sequence obtained in step i. neither matches to a sequence comprised by the decoy sequence database nor to a sequence comprised by the target sequence database, preferably at least one time, before the sequencing of the biopolymer fragment in the nanopore is continued according to step ii. c.

6. Method according to any one of the preceding claims, wherein the one or more target sequences comprise sequences comprised by a pathogen (pathogen sequence), wherein the pathogen is preferably selected from the group of bacteria, viruses, fungi, protists, parasites and/or plasmodia, and/or

wherein the target sequence database comprises one or more pathogen sequences comprising a mutation, a genotype of interest, a resistance, and/or a sensitivity to a certain substance, and/or
wherein the host species is selected from the group of bacterial, viral, fungal, protist, plasmodia, eucaryotic, amphibian, avian, plants, preferably mammal or human.

7. Method according to any one of the proceeding claims, wherein the biopolymer comprising sample is a sample derived from a subject, a patient, a cell culture of patient cells or cell lines, an animal, or a cell culture of animal cells or cell lines, a biopsy, a blood sample, a tissue sample, a urine sample, an environmental sample, or a food-derived sample.

8. Method according to any one of the preceding claims, wherein the biopolymer is selected from a group comprising nucleic acids and polypeptides.

9. Method according to any one of the preceding claims, wherein the decoy sequence database comprises one or more biopolymer sequences representative of a host species, such as genomic nucleic acid sequences or corresponding polypeptide sequences, mitochondrial nucleic acid sequences or corresponding polypeptide sequences, transient nucleic acid sequences or corresponding polypeptide sequences, vector nucleic acid sequences or corresponding polypeptide sequences, or any combination thereof.

10. Method according to any one of the preceding claims, wherein the sequencing in step i. comprises a base calling step, which is achieved by a computational base calling algorithm executed on a Central Processing Unit (CPU), a Graphics Processing Unit (GPU), a Field Programmable Gate Array (FPGA) or a Tensor Processing Unit (TPU) of a computer connected to or comprised within said nanopore sequencing device.

11. Method according any one of the preceding claims, wherein steps i. to ii. are performed in parallel in multiple nanopores of the nanopore sequencing device.

12. Device or system comprising means for performing nanopore sequencing and additional means for performing the method according to claims 1-11, wherein the device or system is preferably a point-of-care (POC) device or system.

13. A computer program [product] comprising instructions which, when executed by a device or system according to claim 12, cause the device or system to carry out steps i. to ii, and optional step iii. of the method according to claims 1-11, and

optionally subsequently causes the device or system to save a final output summary comprising the sequencing information determined in each succession of steps i. to ii and optionally step iii. on a computer-readable storage medium,
wherein, while carrying out steps i. to ii and optionally step iii. of the method, the computer program interacts with the nanopore sequencing device or system to control the sequencing and discarding of the biopolymer at the individual nanopores of the nanopore sequencing device.

14. A computer-readable medium having stored thereon the computer program of claim 13.

15. A computer-implemented method for determining the presence of one or more target sequences in a biopolymer-comprising sample, comprising:

i. receiving sequencing data representing the initial 10-1500 consecutive building blocks of a biopolymer fragment acquired by a nanopore sequencing device during an ongoing nanopore sequencing process,
ii. comparing the biopolymer sequence received in step i. to sequences comprised within a decoy sequence database and/or comparing the biopolymer sequence received in step i. to the one or more target sequences comprised within a target sequence database,
wherein

a. if the sequence received in step i. matches to at least one decoy sequence comprised by the decoy sequence database but not to the sequences comprised by the target sequence database, instructions are sent to the nanopore sequencing device to abort the sequencing of the respective biopolymer fragment and to discard the biopolymer fragment from the nanopore of the nanopore sequencing device, or
b. if the sequence received in step i. matches to at least one target sequence comprised by the target sequence database but not to the sequences comprised by the decoy sequence database,

the presence of the target sequence of the target sequence database with the highest shared sequence identity to the obtained and/or final sequence is determined, and preferably instructions are sent to the nanopore sequencing device to continue the sequencing of the biopolymer fragment in the nanopore of the nanopore sequencing device preferably until the end of the biopolymer fragment is reached and a final sequence is obtained, and preferably
sequencing data from the nanopore sequencing device representing the final sequence of the biopolymer fragment are received,

c. if the sequence received in step i. neither matches to at least one sequence comprised by the decoy sequence database nor to at least one sequence comprised by the target sequence database, or

if the sequence obtained in step i. matches to at least one sequence comprised by both the decoy sequence database and the target sequence database,
instructions are sent to the nanopore sequencing device to continue the sequencing of the biopolymer fragment in the nanopore of the nanopore sequencing device for a defined length of biopolymer, preferably at least 100 building blocks, resulting in an elongated sequence, and
data from the nanopore sequencing device representing the elongated sequence of the biopolymer fragment are received, and
the received elongated sequence is compared to sequences comprised by the decoy sequence database and/or to sequences comprised by the target sequence database according to step ii., wherein one of alternative steps ii. a., b. or c. is performed,

iii. optionally determining the presence of one or more target sequences in the sample according to step ii.b.

16. Data processing apparatus comprising means for carrying out the method of claim 15.

17. A computer program [product] comprising instructions which, when the program is executed by a computer, cause the computer to carry out the method of claim 15.

**Fig. 1**

**Fig. 2**

MinKNOW

*deplete* workflow of the present method

Load index from disk (Interleaved Bloom Filter) → Wait for sequencing to start → Base Calling Queue → Base Calling (DeepNano-blitz) → Length >= 250 bp?

Length >= 250 bp? — No → (back to Base Calling Queue); Yes → Classification Queue → Read Classification → Unblock?

Unblock? — No → Fifth attempt?; Yes → Response Queue

Fifth attempt? — No → Classification Queue; Yes → Response Queue

Start Sequencing

MinION device

send raw signal data

send unblock/continue message

**Fig. 3**

**Fig. 4**

$H_1(\ \boxed{C\ A\ G\ G\ A\ T\ T}\ ) = SV_2 =$

$H_2(\ \boxed{C\ A\ G\ G\ A\ T\ T}\ ) = SV_k = \&\&$

$H_3(\ \boxed{C\ A\ G\ G\ A\ T\ T}\ ) = SV_x = \&\&$

$Count(p) =$

Read $p$

Read $p$ is part of *fragment 2* for threshold $t = 3$

**Fig. 5**

Distribution plots of unblocked read lengths

Distribution of reads across species in simulated mock community dataset

Fig. 6

**Fig. 7**

Fig. 8

Distribution of reads across species in real mock community dataset

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Anonymous: "JensUweUlrich / ReadBouncer (Public)", , 21 January 2022 (2022-01-21), XP055937436, Retrieved from the Internet: URL:https://github.com/JensUweUlrich/ReadBouncer/tree/01d807200f8922701a8f4ef29094c493f2cc60d6 [retrieved on 2022-07-01] * p. 1 and 2, section "Overview", "Installation"; p. 5, section "fragment_size"; p. 9 to 12, sections "Use Cases" and "Test Adaptive Sampling" p. 5, section "kmer size"; * ----- | 1-17 | INV. G16B20/00 G16B40/10 |

TECHNICAL FIELDS
SEARCHED      (IPC)

G16B

The present search report has been drawn up for all claims

| Place of search | Date of completion of the search | Examiner |
|---|---|---|
| Munich | 1 July 2022 | Heidrich, Alexander |

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LOKA et al.** *Bioinformatics,* 15 July 2018, vol. 34 (14), 2376-2383
- **AHMED O ; ROSSI M ; KOVAKA S ; SCHATZ MC ; GAGIE T ; BOUCHER C ; LANGMEAD B.** Pan-genomic Match-ing Statistics for Targeted Nanopore Sequencing. *iScience,* 2021, 102696
- **ANDRUSCH A ; DABROWSKI PW ; KLENNER J ; TAUSCH SH ; KOHL C ; OSMAN AA ; RENARD BY ; NITSCHE A.** PAIPline: pathogen identification in metagenomic and clinical next generation sequencing samples. *Bioinformatics,* 2018, vol. 34, i715-i721
- **BLANCA A ; HARRIS RS ; KOSLICKI D ; MEDVEDEV P.** The statistics of k-mers from a sequence undergoing a simple mutation process without spurious matches. *bioRxiv,* 2021
- **BLOOM BH.** Space/time trade-offs in hash coding with allowable errors. *Communications of the ACM,* 1970, vol. 13, 422-426
- **BOZA V, PERESI~NI P, BREJOVA B, VINAR T.** DeepNano-blitz: a fast base caller for MiniON nanopore sequencers. *Bioinformatics,* 2020, vol. 36, 4191-4192
- **BRINKERHOFF, H. ; KANG, A. S. W. ; LIU, J. ; AK-SIMENTIEV, A. ; DEKKER, C.** Code and Data for "Multiple rereads of single proteins at single-amino acid resolution using nanopores. *Science,* 04 November 2021, vol. 374 (6574), 1509-1513
- **BRINKERHOFF, H. ; KANG, A.S.W. ; LIU, J. ; AK-SIMENTIEV, A. ; DEKKER, C.** Infinite re-reading of single proteins at single-amino-acid resolution using nanopore sequencing. *bioRxiv,* 2021
- **BRODER AZ.** On the resemblance and containment of documents. *Proceedings. Compression and Complexity of SEQUENCES 1997,* 1997, 21-29
- **DADI TH ; SIRAGUSA E ; PIRO VC ; ANDRUSCH A ; SEILER E ; RENARD BY ; REINERT K.** DREAM-Yara: An exact read mapper for very large databases with short update time. *Bioinformatics,* 2018, vol. 34, i766-i772
- **EDWARDS HS ; KRISHNAKUMAR R ; SINHA A ; BIRD SW ; PATEL KD ; BARTSCH MS.** Real-time selective sequencing with RUBRIC: read until with basecall and reference-informed criteria. *Scientific reports,* 2019, vol. 9, 1-11
- **KIETBASA SM ; WAN R ; SATO K ; HORTON P ; FRITH MC.** Adaptive seeds tame genomic sequence comparison. *Genome research,* 2011, vol. 21, 487-493
- **KONO, NOBUAKI ; ARAKAWA, KAZUHARU.** Nanopore sequencing: Review of potential applications in functional genomics. *Development, Growth & Differentiation,* 2019, vol. 61
- **KOSLICKI D ; ZABETI H.** Improving minhash via the containment index with applications to metage-nomic analysis. *Applied Mathematics and Computation,* 2019, vol. 354, 206-215
- **KOVAKA S ; FAN Y ; NI B ; TIMP W ; SCHATZ MC.** Targeted nanopore sequencing by real-time mapping of raw electrical signal with UNCALLED. *Nature Biotechnology,* 2021, vol. 39, 431-441
- **LEGGETT RM ; CLARK MD.** A world of opportunities with nanopore sequencing. *Journal of Experi-mental Botany,* 2017, vol. 68, 5419-5429
- **LI H.** Minimap2: pairwise alignment for nucleotide sequences. *Bioinformatics,* 2018, vol. 34, 3094-3100
- **LOOSE M ; MALLA S ; STOUT M.** Real-time selective sequencing using nanopore technology. *Nature methods,* 2016, vol. 13, 751-754
- **MARTIN S ; HEAVENS D ; LAN Y ; HORSFIELD S ; CLARK MD ; LEGGETT RM.** Nanopore adaptive sampling: a tool for enrichment of low abundance species in metagenomic samples. *bioRxiv,* 2021
- **MIGA KH ; KOREN S ; RHIE A ; VOLLGER MR ; GERSHMAN A ; BZIKADZE A ; BROOKS S ; HOWE E ; PORUBSKY D ; LOGSDON GA et al.** Telomere-to-telomere assembly of a complete human X chromosome. *Nature,* 2020, vol. 585, 79-84
- **MIKHEYEV AS ; TIN MM.** A first look at the Oxford Nanopore MiniON sequencer. *Molecular ecology resources,* 2014, vol. 14, 1097-1102
- **MONGAN AE ; TUDA JSB ; RUNTUWENE LR.** Portable sequencer in the fight against infectious disease. *Journal of human genetics,* 2020, vol. 65, 35-40
- **MOSS EL ; MAGHINI DG ; BHATT AS.** Complete, closed bacterial genomes from microbiomes using nanopore sequencing. *Nature biotechnology,* 2020, vol. 38, 701-707
- **ANDREAS D. BAXEVANIS ; GARY D. BADER ; DAVID S. WISHART.** Bioinformatics. Wiley-Blackwell, 2020
- **NICHOLLS SM ; QUICK JC ; TANG S ; LOMAN NJ.** Ultra-deep, long-read nanopore sequencing of mock microbial community standards. *Gigascience,* 2019, vol. 8, giz043

- **NURK S et al.** The complete sequence of a human genome. *bioRxiv,* 2021
- **ONDOV BD ; TREANGEN TJ ; MELSTED P ; MALLONEE AB ; BERGMAN NH ; KOREN S ; PHILLIPPY AM.** Mash:fast genome and metagenome distance estimation using MinHash. *Genome biology,* 2016, vol. 17, 1-14
- **ONO Y ; ASAI K ; HAMADA M.** PBSIM2: a simulator for long-read sequencers with a novel generative model of quality scores. *Bioinformatics,* 2021, vol. 37, 589-595
- *Nanopore Sequencing Accuracy,* 2020, https://nanoporetech.com/ accuracy
- *Minknow API,* 2021
- **PAYNE A ; HOLMES N ; CLARKE T ; MUNRO R ; DEBEBE BJ ; LOOSE M.** Readfish enables targeted nanopore sequencing of gigabase-sized genomes. *Nature biotechnology,* 2021, vol. 39, 442-450
- **PAYNE A ; HOLMES N ; RAKYAN V ; LOOSE M.** BulkVis: a graphical viewer for Oxford nanopore bulk FAST5 files. *Bioinformatics,* 2019, vol. 35, 2193-2198
- **PIRO VC ; DADI TH ; SEILER E ; REINERT K ; RENARD BY.** ganon: precise metagenomics classification against large and up-to-date sets of reference sequences. *Bioinformatics,* 2020, vol. 36, i12-i20
- **QUICK J ; LOMAN NJ ; DURAFFOUR S ; SIMPSON JT ; SEVERI E ; COWLEY L ; BORE JA ; KOUNDOUNO R ; DUDAS G ; MIKHAIL A et al.** Real-time, portable genome sequencing for Ebola surveillance. *Nature,* 2016, vol. 530, 228-232
- **RANG FJ ; KLOOSTERMAN WP ; RIDDER J.** From squiggle to basepair: computational approaches for improving nanopore sequencing read accuracy. *Genome biology,* 2018, vol. 19, 1-11
- **RUNTUWENE LR ; TUDA JS ; MONGAN AE ; SUZUKI Y.** On-site MiniON sequencing. *Single Molecule and Single Cell Sequencing,* 2019, 143-150
- **SIM J ; CHAPMAN B.** In-field whole genome sequencing using the MiniON nanopore sequencer to detect the presence of high-prized military targets. *Australian Journal of Forensic Sciences,* 2019, vol. 51, S86-S90
- **WICK RR ; JUDD LM ; HOLT KE.** Performance of neural network base calling tools for Oxford Nanopore sequencing. *Genome biology,* 2019, vol. 20, 1-10
- **ZOOK JM ; HANSEN NF ; OLSON ND ; CHAPMAN L ; MULLIKIN JC ; XIAO C ; SHERRY S ; KOREN S ; PHILLIPPY AM ; BOUTROS PC et al.** A robust benchmark for detection of germline large deletions and insertions. *Nature biotechnology,* 2020, 1-9